# EUROPEAN PATENT APPLICATION

(11) **EP 4 410 296 A1**
(43) Date of publication of application: **07.08.2024**
(21) Application number: 22875183.0
(22) Date of filing: 30.09.2022
(51) Int. Cl.: A61K 31/713, A61K 31/551, C07K 14/47, C12N 15/113, A61P 25/00

(54) **DIRECT TRANSDIFFERENTIATION FOR TREATMENT OF NEUROLOGICAL DISEASE**

(30) Priority: 30.09.2021 CN 202111158620
(71) Applicant: Shanghai Genemagic Biosciences Co., Ltd., Shanghai 201108 (CN)
(72) Inventor: ZHOU, Haibo, Shanghai 200031 (CN); HU, Xinde, Shanghai 200031 (CN); SU, Jinlin, Shanghai 200031 (CN)
(74) Representative: Lavoix
(86) International application number: PCT/CN2022/123409
(87) International publication number: WO 2023/051802

(57) **Abstract**

Provided are an RE1/NRSE element as a target for transdifferentiation of non-neuronal cells into neuronal cells, and the use of an RE1/NRSE blocker in the prevention and/or treatment of diseases associated with loss of function or death of neurons. Also provided is a method for blocking an RE1/NRSE element so as to regulate the expression of a neuron-associated gene in non-neuronal cells. The method comprises reducing binding of REST to an RE1/NRSE element or reducing the amount or activity of REST. Also provided is an RE1/NRSE element blocker, particularly a binding structural domain of an endogenous RE1/NRSE binding protein and a variant thereof, which can be used for the prevention and/or treatment of diseases associated with loss of function or death of neurons.

## Description

### TECHNICAL FIELD

The invention relates to the field of biomedicine. More specifically, the present disclosure relates to RE1/NRSE elements as targets for inducing trans-differentiation of non-neuronal cells into neuronal cells; and the use of the RE1/NRSE blockers for the prevention and/or treatment of the diseases associated with neuronal dysfunction or death.

### BACKGROUND

Repressor element 1/neuron-restrictive silencer element (RE1/NRSE) is a specific DNA sequence with a length of about 21bp (vary between 20 and 23bp), RE1/NRSE mainly bind to REST (RE1-silencing transcription factor), which is also known as neuron-restrictive silencer factor (NRSF) and regulate the expression of the gene related to neuron development and maturation. RE1 is a negative regulatory element related to neuron maturation, which was first discovered at the 5' end of the promoters of NaV1.2 and SCG10, and regulates the expression of these genes. In non-neuronal cells, the RE1 site is bound by a silencing complex composed of histone deacetylases and methylases to inhibit the expression of neuron-related genes. However, there are more than 1,800 RE1 elements in mice and humans, making it difficult to regulate with some existing technologies. For example, the technology of CRISPR-mediated gene regulation and epigenetic modification has very high precision and can precisely regulate the expression of specific genes, but it is difficult to regulate the expression of genes regulated by RE1 in this way.

Parkinson's disease (PD) is a disease associated with the loss of neuronal function or the death of neurons, it's characteristic is the loss of dopamine neurons in the substantia nigra of the midbrain. At present, the main methods for treating Parkinson's disease are the use of the small molecule drugs represented by dopamine analogs such as levodopa. Surgery therapy developed in recent years also can improve the symptoms to a certain extent, such as deep brain electrode stimulation. However, these methods can only alleviate the disease partially, but cannot prevent disease progression and slow down the death of dopamine neurons. The trans-differentiation therapy brings hope to the regeneration of dopamine neurons. By overexpressing some genes in glial cells, or gene editing of glial cells, glial cells can be transdifferentiated into dopamine neurons to supplement the missing or dead dopamine neurons. Müller glia (MG) is the main glial cell in retinal tissue. Retinal ganglion cell (RGC) is a nerve cell located in the innermost layer of the retina. Its dendrites mainly establish synaptic connections with bipolar cells, and its axons extend to the optic nerve head to form the optic nerve and extend to brain. Retinal ganglion cell (RGC) damage or degeneration is a major cause of permanent blindness. RGCs are the only output neurons in the retina, so RGC damage or degeneration will lead to permanent blindness. Reconstructing functional RGC is currently the only way to help blind patients restore vision, and RGC cells are very difficult to regenerate. Therefore, exploring how to regenerate RGC cells will bring hope to the majority of blind patients caused by RGC cell death.

Photoreceptor cell death is the main cause of blindness. Photoreceptor cells are divided into rod cells and cone cells. There are many reasons for photoreceptor cell death, including hereditary blindness, acquired blindness and senile degenerative blindness. Common hereditary blindness, such as retinitis pigmentosa and congenital amaurosis, is caused by the death of photoreceptor cells in the retina. In the visual system, photoreceptor cells are the only cells that convert light signals into neural electrical signals. The photoreceptor cells establish a connection with downstream bipolar cells, and transmit the neural electrical signals to bipolar cells. The bipolar cells establish a connection with RGC or amacrine cell, which continue to transmit the neural signals to downstream. In the field of blindness caused by the death of photoreceptor cells, there is no drug available for treatment currently. With the development of gene therapy technology, drugs for blindness caused by genetic factors have gradually been developed, but such drugs can only be used for hereditary blindness in which photoreceptor cells have not died, such as the gene therapy drug developed by Spark to treat RPE65 gene dysfunction. Currently, there is no drug for treating blindness patients whose photoreceptor cells have died. In this field, Hopes have been placed on the stem cell therapy and regenerative therapy.

The death of spiral ganglion cells in the inner ear is the main cause of nerve deafness. Inner ear spiral ganglion cells are located in the inner ear spiral ganglion, one end of which is connected to the inner ear hair cells, and the other end is connected to the central nervous system. It is the only channel for transmitting auditory signals to the central nervous system. Inner ear spiral ganglion cells are a very special type of neurons, and their gene expression profiles are very different from ordinary neurons. Permanent deafness caused by the death of spiral ganglion cells in the inner ear, no matter whether it is hereditary or non-hereditary, there is no drug available for treatment currently. Due to the special structure of the inner ear spiral ganglion, stem cell transplantation technology has not yet achieved progress. Another technology that is highly anticipated is in situ regeneration technology, but due to the particularity of the inner ear spiral ganglion cells, the inner ear spiral ganglion cells have not been successfully induced so far. Although some studies have induced inner ear spiral ganglion cells through transgenic mice, they are produced during the developmental process. The deafness caused by the death of inner ear spiral ganglion cells often occurs in adults or the elderly, while in the mature inner ear system Inner ear spiral ganglion cells have never been induced so far.

In previous studies, scientists successfully induced a variety of special types of neurons in the in vitro culture system by adding different media and small molecules, and at the same time coordinating the expression of multiple genes and transcription factors. But these in vitro experiments are difficult to apply in vivo. The complex environment in the body leads to the fact that various factors screened in vitro failed to function in vivo as in vitro experiments. Therefore, many factors that induce neuronal trans-differentiation screened in vitro cannot induce glial cell trans-differentiation into neurons in vivo. At present, the factors that can successfully induce neurons in vivo are mainly AscL1, NgN2, NeuroD1 and PTBP1. Although AscL1, NgN2 and NeuroD1 can induce neurons in vivo, they can only produce ordinary glutamatergic neurons, but cannot induce neurons with special functions, such as: dopamine neurons, serotonergic neurons, cholinergic neurons, retinal ganglion cells, photoreceptor cells and cochlear spiral ganglion cells, etc. Among the trans-differentiation factors reported so far, only Ptbp1 can induce glial cells to transdifferentiate into a special type of neuron in vivo, and its type is dopamine neuron. Although it has also been reported that the simultaneous overexpression of AscL1+NeuroD1+Lmx1b+miR218 in glial cells can promote glial cells to produce dopamine neurons, but its efficiency is very low, and it is limited by the co-infection of multiple AAVs. It is almost impossible to achieve in drug development. This also shows that it is a challenging research task to realize the regeneration of special types of neurons in vivo, but it has an important meaning to regenerate special types of neurons. These special types of neurons are often the most dominant cell type in diseases faced by humans. As mentioned above, these cells are related to many diseases. Currently, both stem cell transplantation technology and trans-differentiation regenerative technology still need to make breakthroughs. Therefore, there is an urgent need in the art to develop methods capable of regenerating dopamine neurons, retinal ganglion cells, photoreceptor cells or other functional nerves.

### SUMMARY OF THE INVENTION

In one aspect, the present application provides a method for blocking RE1/NRSE element to regulate the expression of neuron-related genes in non-neuronal cells, which includes reducing the binding of REST and RE1/NRSE element, or reducing the amount or activity of REST.

In some embodiments, the amount of REST is reduced by methods such as gene editing, small RNA interference, or accelerated protein degradation.

In some embodiments, the amount of REST is reduced by methods such as gene editing, antisense oligonucleotides (ASO), small RNA interference, miRNA technology, small molecule compounds, or accelerated protein degradation.

In some embodiments, the activity of REST is reduced by removing the inhibitory region of REST through gene editing or by giving a REST inhibitor.

In some embodiments, the binding of REST to RE1/NRSE element is blocked by REST-binding agent, such as a REST antibody.

In some embodiments, the binding of REST to the RE1/NRSE element is blocked by the binding of a RE1/NRSE element blocker to the RE1/NRSE element.

In some embodiments, the RE1/NRSE element blocker is a REST competitive binding protein, short peptide or gene editing protein or the encoding nucleic acid thereof, or nucleic acid and nucleic acid analogs, or a small molecule RE1/NRSE element blocker.

In some embodiments, the RE1/NRSE element blocker is a REST variant or nucleic acid encoding it.

In some embodiments, the REST variant is the DNA binding domain of REST, which lacks the N-terminal and C-terminal inhibitory domains of REST, preferably contains amino acids from 155 to 420 of REST.

In some embodiments, the DNA binding domain of the REST is fused to an activation domain.

In some embodiments, the activation domain is selected from: epigenetic modification proteins or gene activation regulatory elements, such as VP64, P65-HSF1, VP16, RTA, Suntag, P300, CBP or combinations thereof, preferably selected from VP64 or P65-HSF1.

In some embodiments, the REST variant comprises the amino acid sequence of SEQ ID NO: 1, 3, 5 and 9 or the nucleotide sequence of SEQ ID NO: 2, 4, 6 and 10, or at least 70%, 60%, 50% identity percentage with anyone thereof.

In some embodiments, the non-neuronal cells include, for example, glial cells, fibroblasts, stem cells, neural precursor cells, neural stem cells, wherein glial cells are selected from astrocytes, oligodendrocytes glial cells, ependymal cells, Schwann cells, NG2 cells, satellite cells, Müller glial cells, inner ear glial cells or combinations thereof, preferably selected from astrocytes, Müller glial cells and cochlear glia cell.

In some embodiments, the glial cells are derived from the brain, spinal cord, eyes or ears, wherein the glial cells in the brain are derived from the striatum, the substantia nigra, the ventral tegmental area of the midbrain, the spinal cord, the hypothalamus, dorsal midbrain or cerebral cortex, preferably derived from striatum and substantia nigra.

In some embodiments, modulating the expression of neuron-associated genes in the non-neuronal cells, so that the non-neuronal cells are transdifferentiated into neuronal cells, wherein the neuronal cells are mammalian neurons , wherein preferred are dopamine neurons, GABA neurons, 5-HT neurons, glutamatergic neurons, ChAT neurons, NE neurons, motor neurons, spinal cord neurons, spinal cord motor neurons, spinal cord sensory neurons, photoreceptors (rods and cones), bipolar cells, horizontal cells, amacrine cells, retinal ganglion cells (RGCs), cochlear nerve cells (cochlear spiral ganglion cells and vestibular neurons), pyramidal nerves Neurons, interneurons, medium spiny neurons (MSN), Purkinje cells, granule cells, olfactory sensory neurons, peribulbar cells or combinations thereof, more preferred are dopamine neurons, retinal ganglion cells, photoreceptor cells and cochlea Spiral ganglion cells.

In some embodiments, the non-neuronal and/or neuronal cells are from, for example, humans, non-human primates, rats and mice, preferably from humans.

In another aspect, the present application provides a use of a RE1/NRSE element blocker for the preparation of medicines for the prevention and/or treatment of diseases associated with neuronal dysfunction or death, wherein the RE1/NRSE element blocker reduces the binding of RE1/NRSE endogenous binding factors to RE1/NRSE elements, wherein the RE1/NRSE endogenous binding factors include zinc finger proteins such as REST.

In some embodiments, the RE1/NRSE element blocker binds to the RE1/NRSE element so as to block the binding of the RE1/NRSE endogenous binding factor to the RE1/NRSE element.

In some embodiments, the RE1/NRSE element blocker is a REST competitive binding protein, short peptide or gene editing protein or its encoding nucleic acid, or nucleic acid and nucleic acid analogs, or a small molecule RE1/NRSE element blocker.

In some embodiments, the RE1/NRSE element blocker is a REST variant or nucleic acid encoding it.

In some embodiments, the REST variant is the DNA binding domain of REST, which lacks the N-terminal and C-terminal repression domains of REST, preferably contains amino acids from positions 155 to 420of REST.

In some embodiments, the DNA binding domain of the REST is fused to an activation domain.

In some embodiments, the activation domain is selected from: an epigenetic modification protein or a gene activation regulatory element, such as VP64, P65-HSF1, VP16, RTA, Suntag, P300, CBP or a combination thereof, preferably selected from VP64 or P65-HSF1.

In some embodiments, the REST variant comprises the amino acid sequence of SEQ ID NO: 1, 3, 5 and 9 or the nucleotide sequence of SEQ ID NO: 2, 4, 6 and 10, or comprises the sequence which has at least 70%, 60%, or 50% identity percentage with anyone thereof.

In some embodiments, the disease associated with neuronal dysfunction or death is selected from: Parkinson's disease, Alzheimer's disease, stroke (stroke), schizophrenia, Huntington's disease, depression, motor neuron disease, amyotrophic lateral sclerosis, spinal muscular atrophy, Pick disease, sleep disorders, epilepsy, ataxia, visual impairment due to RGC cell death, glaucoma, age-related RGC lesions, optic nerve damage, retinal ischemia or hemorrhage, Leber hereditary optic neuropathy, degeneration or death of photoreceptor cells due to damage or degeneration, macular degeneration, retinitis pigmentosa, diabetes-related blindness, night blindness, color blindness, hereditary blindness, amaurosis, deafness or hearing loss due to spiral ganglion cell death, or a combination thereof.

In another aspect, the present application provides a RE1/NRSE element blocker, which is a REST variant or its encoding nucleic acid, or an artificially designed analog of the DNA binding domain of REST.

In some embodiments, the REST variant is the DNA binding domain of REST, which lacks the N-terminal and C-terminal repression domains of REST, preferably contains amino acids from 155 to 420 of REST.

In some embodiments, the DNA binding domain of the REST is fused to an activation domain.

In some embodiments, the activation domain is selected from: epigenetic modification proteins or gene activation regulatory elements, such as VP64, P65-HSF1, VP16, RTA, Suntag, P300, CBP or combinations thereof, preferably selected from VP64 or P65-HSF1.

In some embodiments, the REST variant comprises the amino acid sequence of SEQ ID NO: 1, 3, 5 and 9 or the nucleotide sequence of SEQ ID NO: 2, 4, 6 and 10, or comprises at least 70%, 60%, or 50% identity percentage with anyone thereof.

In some embodiments, the REST variant or the DNA binding domain of REST is from, for example, a human, non-human primate, rat and mouse, preferably from a human.

In another aspect, the present application provides a pharmaceutical composition or medicine box or kit, which comprises the above blocker.

In some embodiments, the pharmaceutical composition or medicine box or kit is formulated for injection, intracranial administration, intraocular administration, intraaural administration, inhalation, parenteral administration, intravenous administration, intramuscular, intradermal, topical administration or oral administration.

In some embodiments, the pharmaceutical composition or medicine box or kit further comprises a vector or carrier for delivering the RE1/NRSE element blocker, wherein the vector or carrier is a viral vector, liposomes, nanoparticles, exosomes, virus-like particles, wherein the viral vectors include recombinant adeno-associated viral vectors (rAAV), adeno-associated viral (AAV) vectors, adenoviral vectors, lentiviral vectors, retroviral vectors, poxvirus vectors, herpes virus vectors, SV40 virus vectors, and combinations thereof, wherein AAV and rAAV are preferred.

In some embodiments, the pharmaceutical composition or medicine box or kit comprises an expression vector for expressing a REST variant, wherein the expression vector comprises a nucleotide sequence encoding a REST variant, which is operably linked to a the promoter induced its expression.

In some embodiments, the pharmaceutical composition or medicine box or kit is used for topical administration to at least one of the following: 1) glial cells in the striatum; ii) glial cells in the substantia nigra of the brain iii) glial cells in the retina; iv) glial cells in the inner ear; v) glial cells in the spinal cord; vi) glial cells in the prefrontal cortex; vii) glial cells in the motor cortex; viii) glial cells in the hypothalamus; and ix) glial cells in the ventral tegmental area (VTA).

In some embodiments, the pharmaceutical composition or medicine box or kit further comprises i) one or more dopamine neuron-related factors, or ii) one or more retinal ganglion cell-associated factors used to express in Müller glial cells,
1) the one or more dopamine neuron-related factors are selected from: FoxA2, Lmx1a, Lmx1b, Nurr1, Pbx1a, Pitx3, Gata2, Gata3, FGF8, BMP, En1, En2, PET1, Pax family protein (Pax3, Pax6, etc.), SHH, Wnt family proteins and TGF-β family proteins, or combinations thereof;
2) the one or more retinal ganglion cell-related factors include: β-catenin, Oct4, Sox2, Klf4, Crx, aCamKII, Brn3a, Brn3b, Brn3C, Math5, Otx2, Ngn2, Ngn1, AscL1, miRNA9, miRNA-124, Nr2e3 and Nrl, and the like.

In some embodiments, the promoter is a glial cell-specific promoter or a Müller glial cell (MG) cell-specific promoter, and the glial cell-specific promoter is selected from: GFAP promoter, ALDH1L1 promoter, EAAT1/GLAST promoter, glutamine synthetase promoter, S100β promoter EAAT2/GLT-1 promoter and Rlbp1 promoter, preferably selected from GFAP promoter.

In some embodiments, the glial cells have a trans-differentiation efficiency of at least 1%, or at least 10%, 20%, 30%, 40%, or 50%.

### DESCRIPTION OF FIGURES

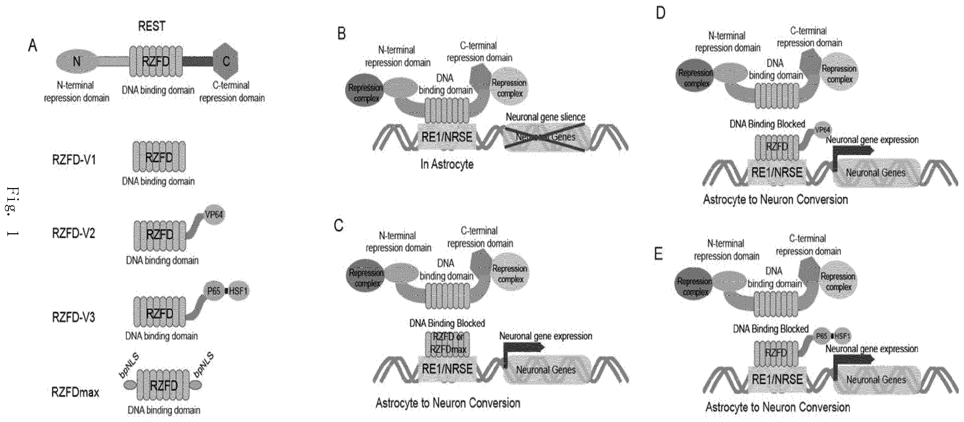

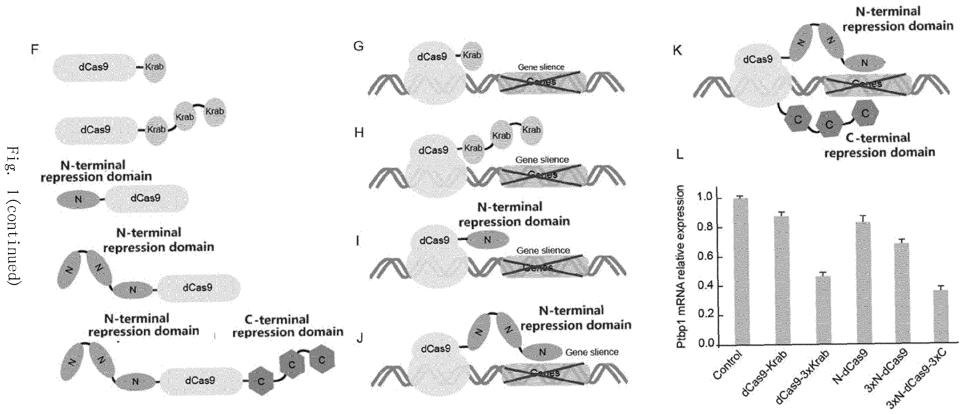

Fig. 1 Schematic diagram of endogenous zinc finger proteins and suppression system. (A) Schematic diagram of the REST protein. The REST protein comprises an N-terminal repression domain, a DNA-binding structure in the middle responsible for binding to RE1, and a C-terminal transcription repression domain. RZFD-V1 represents the first design of RZFD, which contains eight zinc finger domains (RZFD, REST Zinc Figure domain) in the middle of human REST protein responsible for binding to RE1 DNA. In the two designs of RZFD-V2 and RZFD-V3, we fused two different activators with RZFD, and named them RZFD-V2 and RZFD-V3, respectively. RZFD-V2 is obtained by fusing the C-terminus of RZFD with VP64, and RZFD-V3 is composed of a transcriptional activation domain of P65 and HSF1 fused in the C-terminus of RZFD. RZFDmax optimizes the codon of RZFD to increase its expression in mammals, and at the same time adds BPNLS sequences at both ends of RZFD to enhance its efficiency entering into nucleus and performing a function. (B) In glial cells, REST binds to RE1, and the C-terminal and N-terminal of REST respectively recruit some transcriptional repressors, so that the neuron-related genes regulated by RE1 cannot be expressed. (C) We imagined that after expressing RZFD-V1 in glial cells, RZFD would binding to RE1 competitively, preventing the binding of REST and RE1, so that the REST silencing complex could not be formed and the genes including neuron-associated gene regulated by RE1 could not be inhibited. (D) RZFD-V2 competitively binds to RE1 through the RZFD domain, prevents the binding of REST/NRSF and RE1, and the VP64 domain can recruit transcriptional activators to enhance the expression of neuron-related genes regulated by RE1. (E) During the trans-differentiation of glial cells into neurons, the RZFD domain of RZFD-V3 binds to RE1, prevents the binding of REST and RE1, disinhibit the repression of REST to the genes regulated by RE1, and at the same time P65-HSF1 activates the domain to recruit some transcriptional activators, which further promote the expression of neuron-related genes regulated by RE1. (F) Schematic diagram of the inhibition system designed by the N-terminal and C-terminal of REST, using dCas9 to connect with the N-terminal and C-terminal of REST, respectively, dCas9-Krab is the positive control system. (G and H) Schematic diagram of dCas9-Krab or dCas9-3xKrab inhibiting the expression of target genes under the action of gRNA. (I-K) Schematic diagram of N-dCas9, 3xN-dCas9 or 3xN-dCas9-3xC inhibiting target gene expression under the guidance of gRNA, wherein N represents the N-terminal inhibitory domain of REST, and C represents the C-terminal inhibitory domain of REST. (L) The expression efficiency of Ptbp1 gene which inhibited by the HEK293T cells transfected by various suppression systems. The inhibition efficiency of dCas9-Krab, N-dCas9 and 3xN-dCas9 was relatively low, and the inhibition efficiency of dCas9-3xKrab and 3xN-dCas9-3xC was relatively high.

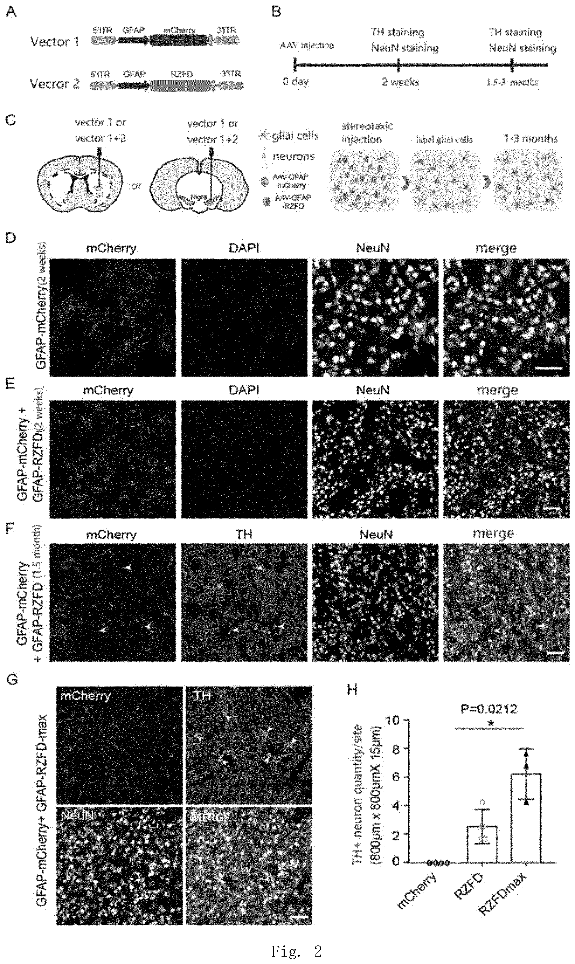

Fig. 2 RZFD-mediated trans-differentiation of glial cells into neurons. (A) Schematic diagram of AAV vector. Vector 1 is a vector schematic diagram of GFAP-driven expression of mCherry. GFAP is a promoter specifically expressed in glial cells. mCherry is a red fluorescent protein used to label glial cells. Vector 2 is a schematic diagram of the human RZFD expression vector, and the expression of RZFD is driven by the astrocyte-specific promoter GFAP. (B) Schematic diagram of injection and sample analysis. The day of AAV injection was recorded as day 0. The results of trans-differentiation of glial cells into neurons were analyzed 2 weeks after injection, and the result of trans-differentiation of glial cells into dopamine neurons was analyzed in 1.5 months after injection. (C) Schematic diagram of AAV virus injection and trans-differentiation. Inject GFAP-mCherry alone in the striatum or substantia nigra of mice, or inject a mixed AAV of GFAP-mCherry and GFAP-RZFD. GFAP-mCherry will label glial cells as red, while GFAP-RZFD trans-differentiates glial cells into neuronal neurons. (D) After GFAP-mCherry injection alone in the striatum of wild-type C57 mice, astrocytes are marked in red, the mCherry channel indicates astrocytes labeled by the GFAP-mCherry, and the nucleus is stained by DAPI, NeuN is a neuron-specific marker, and the merge diagram shows the GFAP-mCherry specifically labeled astrocytes but not neurons. (E) Two weeks after injection of mixed AAV of GFAP-mCherry and GFAP-RZFD in the striatum of mice, most glial cells have begun to deform, and a small number of mCherry-positive cells have begun to express NeuN, but no cells Express TH, wherein NeuN is a neuron-specific marker, and TH is a dopamine neuron-specific marker. (F) In 1.5 months after injection of mixed AAV of GFAP-mCherry and GFAP-RZFD in the striatum of mice, most of the mCherry-positive cells expressed neuron-specific markers, and some cells express dopamine specific marker TH. Arrows indicate the neuron co-expressing mCherry and TH. (G) About 1.5 months after injection of mixed AAV of GFAP-mCherry and GFAP-RZFDmax in the striatum of mice, GFAP-mCherry + GFAP-RZFDmax promoted the trans-differentiation of astrocytes in the striatum of mice into dopamine neurons, TH is a specific marker of dopamine. The scale bar is 50 microns. (H) Quantity statistics of dopamine neurons produced by RZFD and RZFDmax. There were no TH positive cells in the control group, and the number of dopamine neurons produced in RZFDmax group was significantly (P=0.0212) more than that of the RZFD group.

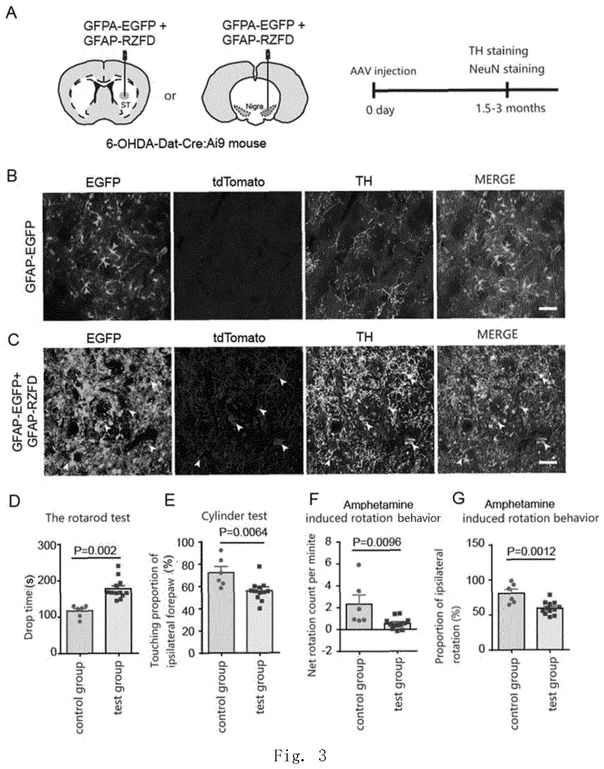

Fig. 3 RZFD transdifferentiates astrocytes into dopamine neurons in DAT-Cre: Ai9 mice modeled with 6-OHDA. (A) Schematic diagram of AAV injection. GFAP-EGFP labeled AAV, under the activation of the GFAP promoter, GFAP-EGFP specifically labeled astrocytes. The mixture of GFAP-EGFP and GFAP-RZFD was injected into the striatum or substantia nigra of mice to induce the trans-differentiation of astrocytes into dopamine neurons. (B) Analysis after AAV injection in the control group, the green fluorescent signal is the astrocytes specifically labeled by GFAP-EGFP, which still presented a typical astrocyte morphology, there were no tdTomato signal of dopamine neurons, and there were no TH-positive cells, the yellow arrows point to EGFP-positive astrocytes. (C) In the group injecting the mixture of GFAP-EGFP and GFAP-RZFD, almost all green fluorescently labeled cells show neuron morphology, tdTomato signal appeared in striatum, tdTomato signal was co-labeled with TH signal, the white arrow points to TH positive tdTomato red cells. The scale bar is 50 microns. (D) The rotarod test was performed on the mice in the GFAP-EGFP-injected control group and the GFAP-EGFP + GFAP-RZFD co-injected test group, there were more than 8 mice in each group. (E) Cylinder test, counting the number of times the front paw touched the wall on the same side of the body, and there were more than 8 mice in each group. (F and G) Drug-induced rotation behavior test, counting the number of rotations per minute and the proportion of rotations on the same side, there were more than 8 mice in each group.

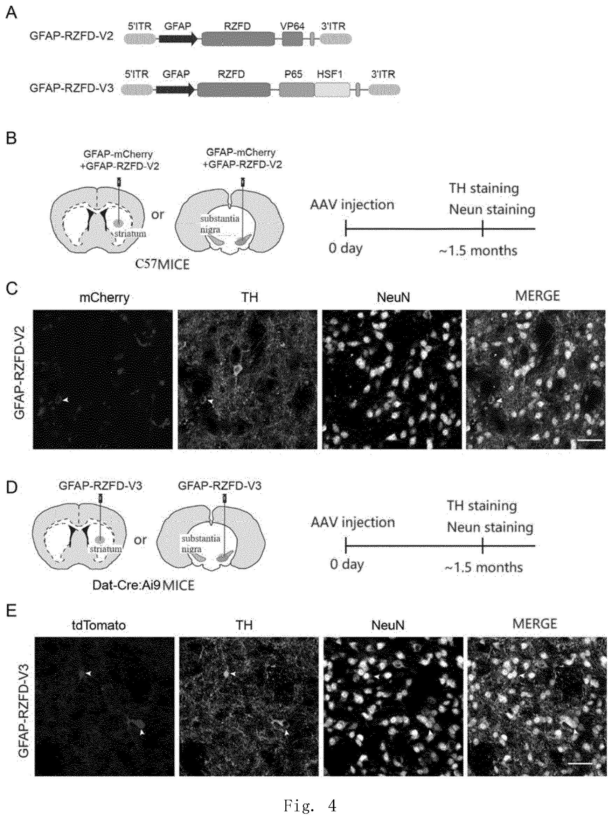

Fig. 4 Trans-differentiation of glial cells into neurons mediated by RZFD-VP64 or RZFD-P65-HSF1. (A)Vectors schematic diagram of GFAP-RZFD-V2 and GFAP-RZFD-V3. RZFD-V2 is a fusion protein of RZFD and VP64 drived by the glial cell-specific promoter GFAP. RZFD-V3 is composed of RZFD and P65-HSF1 activation domain. (B) Schematic diagram of striatum or substantia nigra of mice injected with the AAV mixture of GFAP-RZFD-V2 and GFAP-mCherry, and analyzed in 1.5 months after injection. (C) Representative diagram of GFAP-RZFD-V2 transdifferentiating glial cells into neurons or dopamine neurons in the striatum, mCherry is the cells labeled by GFAP-mCherry, TH is the specific marker of dopamine neurons, NeuN is a neuron-specific marker. Arrows indicate neurons co-expressing both mCherry and TH. (D) Schematic diagram of injecting GFAP-RZFD-V3 in DAT-Cre : Ai9 mice, GFAP-RZFD-V3 was injected into the striatum or substantia nigra of DAT-Cre : Ai9 mice, sacrificed and analysis in 1.5 months after injection, Dat -Cre is a product inserted Cre behind the Dat's endogenous promoter, Ai9 is Rosa26-CAG-LSL-tdTomato-WPRE mice, DAT-Cre: only mature dopamine neurons in the brain of Ai9 mice can be labeled by tdTomato red fluorescent signal. (E) Representative image of analysis results obtained in 1.5 months after injection of GFAP-RZFD-V3 in the striatum, the red fluorescent signal (tdTomato) represents the transdifferentiated mature dopamine neurons, and the green signal is TH staining which is the specific marker of dopamine neurons, the white signal is NeuN staining which is the neuron-specific marker, the Merge diagram shows that the red signal of tdTomato overlaps with the green signal of TH staining, and the arrows indicate the neurons co-expressing both tdTomato and TH. The scale bar is 50 microns.

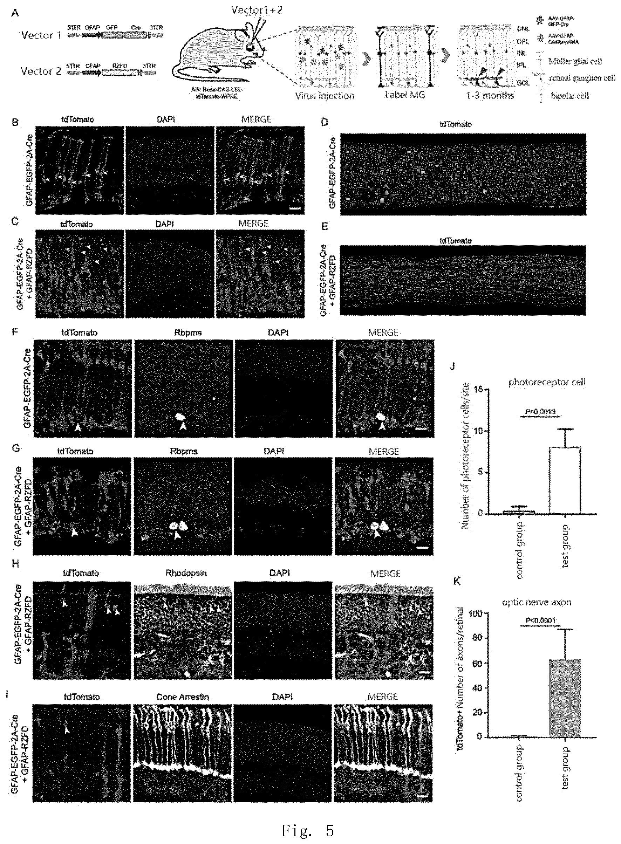

Fig. 5 Overexpression of GFAP-RZFD in the retina transdifferentiates Müller glia cells into photoreceptor cells or retinal ganglion cells. (A) Schematic diagram of overexpressing GFAP-RZFD to transdifferentiate Müller glial cells into photoreceptor cells or retinal ganglion cells. Vector 1 is GFAP-EGFP-2A-Cre, which is injected into the retina of Ai9 mice and can be used for specifically labelling Müller glial cells in mice, and the labeled cells will be labeled with tdTomato. (B) The red fluorescent signal was specifically expressed in Müller glial cells after the control virus GFAP-EGFP-2A-Cre was injected into the retina of Ai9 mice. (C) In the group injected with GFAP-RZFD + GFAP-EGFP-2A-Cre, the white arrow points to the photoreceptor cells from trans-differentiation. The scale bar is 50 microns. (D) The optic nerve of mice injected with GFAP-EGFP-2A-Cre virus in the control group, there was no tdTomato-labeled axons to be observed in the optic nerve. (E) A large number of tdTomato-labeled axons were observed in the optic nerve of mice injected with GFAP-RZFD + GFAP-EGFP-2A-Cre. (F) The red fluorescent signal was specifically expressed in Müller glial cells after the subretinal injection of control virus GFAP-EGFP-2A-Cre in the NMDA-modeled Ai9 mice, and it was not co-labeled with the RGC-specific protein marker Rbpms, yellow arrows point to RGCs positive for Rbpms. (G) In the retina injected with AAV in the GFAP-RZFD group, some tdTomato-positive cells were found in the retinal ganglion cell layer, and were co-labeled with the RGC-specific protein marker Rbpms, the white arrows pointed to the cells that were positive for both tdTomato and Rbpms. (H) Staining of the photoreceptor cell-specific protein marker Rhodopsin, some tdTomato-positive cells were co-labeled with Rhodopsin in the retinal outer granular layer of the GFAP-RZFD-injected group, the white arrows pointed to the co-labeled cells. (I) Cone Arrestin (mCAR) staining, a small number of tdTomato-positive cells were co-labeled with mCAR in the retinal outer granular layer of the GFAP-RZFD-injected group, and the white arrows pointed to the co-labeled cells. The scale bar is 50 microns. (J) The number of tdTomato-positive photoreceptor cells in the control group and the test group was counted. There were almost no tdTomato-labeled photoreceptor cells in the control group, and tdTomato-positive photoreceptor cells were observed in the GFAP-RZFD overexpression group. Each group had more than 3 mice. (K) The number of tdTomato-positive axons in the optic nerve of mice injected with GFAP-EGFP-2A-Cre control AAV and GFAP-RZFD + GFAP-EGFP-2A-Cre group AAV, each group had more than 3 mice.

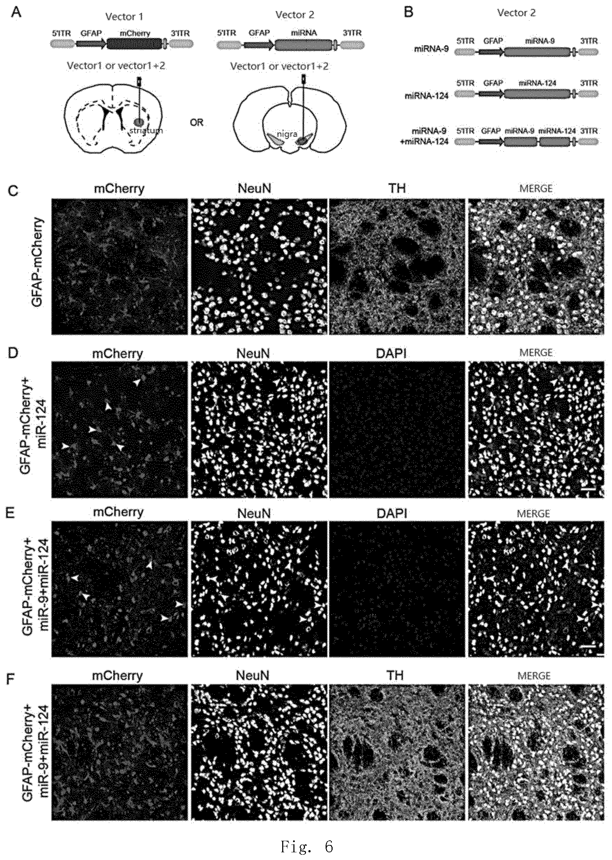

Fig. 6 Overexpression of miRNA can transdifferentiate mouse astrocytes into neurons but not dopamine neurons. (A) Schematic illustration of the conversion of astrocytes into neurons through miRNAs overexpression. Vector 1 is AAV-GFAP-mCherry, and vector 2 (AAV-GFAP-miRNA) is driven by GFAP to overexpress miRNA. The control group was injected with Vector 1 alone, the test group was injected with vector 1+2. (B) Vector 2 corresponds to figure A. Schematic diagram of the AAV vector expressing miR-124 (comprising miR-124-5p and miR-124-3p), miR-9 (comprising miR-9-5p and miR-9-3p) or miR-9-miR-124 driven by the GFAP promoter. (C) Injection of GFAP-mCherry in the striatum of mice, mCherry expresses in glial cells specifically, and there is no TH positive cell in the striatum. (D) The test group AAV virus (GFAP-mCherry + GFAP-miR-124) was injected into the mouse striatum, the white arrow points to the labeled cells, the superimposed image shows that the red fluorescence does not overlap with NeuN, the scale bar is 50 microns. (E) Injection of GFAP-mCherry + GFAP-miR-9+miR-124 in the striatum of mice, the white arrows point to the labeled cells, the superimposed image shows that the red fluorescence overlaps with NeuN, the scale bar is 50 µm. (F) Injection of GFAP-mCherry + GFAP-miR-9+miR-124 in the striatum of mice, TH is a specific marker for dopamine neurons, the white arrow points to the marked cells, and the merge diagram shows that the red fluorescence does not overlap with and TH signals, the scale bar is 50 µm.

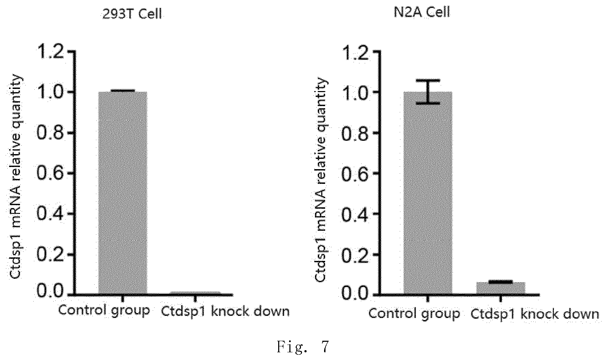

Fig. 7 CasRx can knock down the mRNA expression of Ctdsp1 in vitro. mRNA of Ctdsp1 could be knocked down when the mRNA of CasRx and a gRNA targeting Ctdsp1 expressed in human 293T or mouse N2A cells Lines in vitro. The full name of Ctdsp1 is Carboxy-terminal domain RNA polymerase II polypeptide A small phosphatase 1 (Ctdsp1).

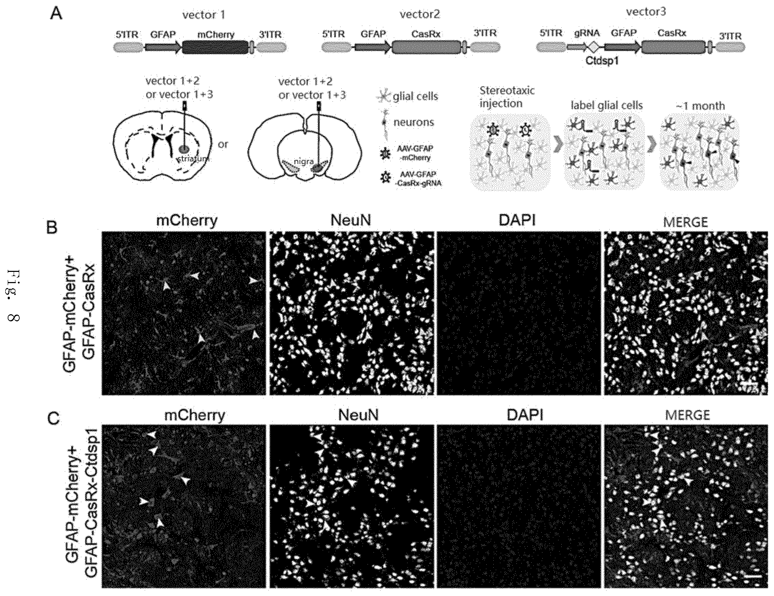

Fig. 8 Knockdown of Ctdsp1 can transdifferentiate mouse astrocytes into neurons but not dopamine neurons. (A) Schematic of astrocyte-to-neuron conversion via gene knockdown. Vector 1 (AAV-GFAP-mCherry) was driven to express fluorescent protein mCherry by the astrocyte-specific promoter GFAP, and vector 2 (AAV-GFAP-CasRx) was was driven to express RNA editing protein CasRxby GFAP. Vector 3 (AAV-GFAP-CasRx-Ctdsp1) encodes CasRx and a gRNA targeting Ctdsp1. C57BL/6 mice were injected with AAV-GFAP-CasRx-Ctdsp1 or control vectors AAV-GFAP-CasRx and AAV-GFAP-mCherry without gRNA into the striatum or substantia nigra. AAV-GFAP-mCherry co-injection was used to specifically label astrocytes or neurons transformed from astrocytes. Testing the transformation in 1-2 months after injection. Carrier 1+2 is the control group, and carrier 1+3 is the test group. (B) The control group AAV (GFAP-mCherry + GFAP-CasRx) was injected into the striatum of mice, the orange arrows point to the labeled astrocytes, NeuN is a neuron-specific marker, and the superimposed figure shows red Fluorescence does not overlap with NeuN, the bar is 50 µm. (C) Trans-differentiation results of glial cells into neurons after injection of the experimental group AAV virus (GFAP-mCherry + GFAP-CasRx-Ctdsp1) in the striatum of mice, the white arrows point to the cells co-labeled with the red fluorescent signal and NeuN, the scale bar is 50 microns.

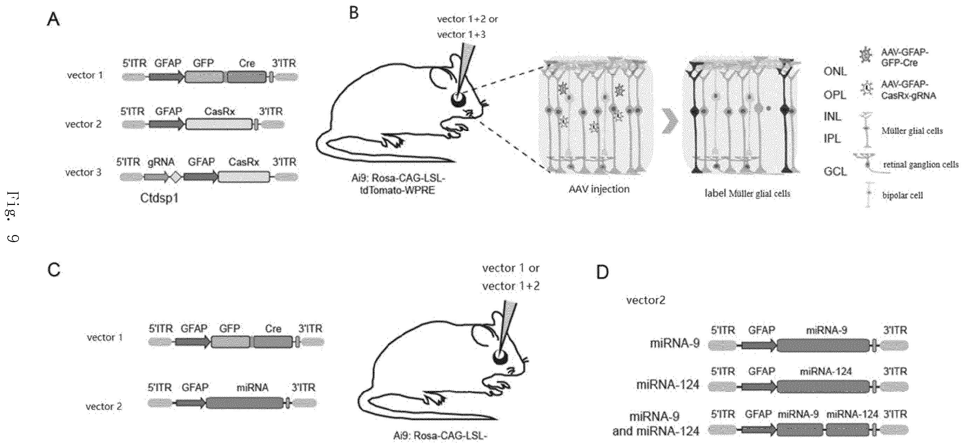

Fig. 9 Knockdown of Ctdsp1 or overexpression of miRNA fails to generate retinal ganglion cells, photoreceptor cells, or cochlear spiral ganglion cells. (A) Schematic diagram of vector design, vector 1 (GFAP-EGFP-2A-Cre) is driven by the astrocyte-specific promoter GFAP to specifically express Cre in Müller glial cells, and vector 2 (GFAP-CasRx) is driven by GFAP to express the RNA editing protein CasRx. Vector 3 (GFAP-CasRx-Ctdsp1) encodes CasRx and a gRNA targeting Ctdsp1. (B) GFAP-CasRx-Ctdsp1 + GFAP-EGFP-2A-Cre or control virus GFAP-CasRx + GFAP-EGFP-2A-Cre were injected into mouse retina. GFAP-EGFP-2A-Cre co-injection was used to specifically label Müller glia. Vactor 1+2 is the control group, and vector 1+3 is the test group. (C) Schematic of miRNA overexpression in retina. Vector 1 is GFAP-EGFP-2A-Cre, and vector 2 (GFAP-miRNA) is driven by GFAP to overexpress miRNA. Vector 1 was injected alone as the control group, and vector 1+2 was injected as the test group. (D) Corresponding to vector 2 in Figure C. Schematic diagram of AAV expression vectors driven by GFAP promoter to express miR-124, miR-9 or miR-9-miR-124, respectively.

### SPECIFIC IMPLEMENTATIONS

The present application provides compositions related to the regulation of RE1/NRSE, biologically active molecules modified based on different domains of the endogenous RE1/NRSE binding protein REST, and applications thereof. In particular, the present application relates to the modification of the endogenous RE1-binding protein REST, so as to utilize the function of different regions in the REST protein to regulate gene expression.

Repressor element 1/neuron-restrictive silencer element (repressor element 1/neuron-restrictive silencer element, RE1/NRSE) is a specific DNA sequence with a length of about 21bp (ranging from 20-23bp), present in many promoter regions of neural-related genes. In the present application, RE1/NRSE and RE1 are used interchangeably. RE1 is a negative regulatory element related to neuron maturation, which was first discovered at the 5' end of the promoter of NaV1.2 and SCG10, and regulates the expression of these genes. In non-neuronal cells, the RE1 site is bound by a silencing complex composed of histone deacetylases and methylases to inhibit the expression of neuron-related genes. There are more than 1800 RE1 elements in mice and humans.

RE1 mainly binds to REST (RE1-silencing transcription factor) to regulate gene expression related to neuronal development and maturation. REST, also known as neuron-restrictive silencer factor (NRSF), is an endogenous protein that binds to RE1. Please refer to SEQ ID NO: 20 for the full-length sequence of the human REST protein.

Through domain prediction and protein structure modeling, the inventors of the present application find that the positions from 159 to 412 of human REST protein contains eight zinc finger domains (ZFD) (Fig. 1A). The positions from 1 to 83 in N-terminal of REST is its N-terminal inhibitory region, which mainly binds to the proteins such as Sin3a and Sin3b, the positions from 1008 to 1097 is its C-terminal inhibitory domain and a zinc finger domain, which mainly binds to the proteins such as RCOR1. The positions from 84 to 158 and from 413 to 1007 in the middle have no obvious protein domains, and their functions are not yet clear, which may be involved in regulating the binding of REST and RE1. The zinc finger domain in the REST protein may be related to its binding to RE1, thereby allowing the transcriptional repression domain in the REST protein (thought to exist at the N-terminal or C-terminal of the REST protein) to regulate the transcription of RE1, thereby inhibiting the RE1 target gene expression. Previous studies have shown that deleting positions from 1 to 83 and from 1008 to 1097 of the REST protein does not affect the binding of REST to RE1, but cannot function normally.

In one aspect, the present application provides a method for transdifferentiating non-neuronal cells into functional neurons in an individual by modulating RE1. In certain embodiments, by blocking REST binding to RE1/NRSE elements at a site of interest in vivo (e.g., a site affected by a disease), trans-differentiation of non-neuronal cells into functional neurons can be achieved at the interest site.

Although previous studies have shown that RE1 exists in the promoter regions of many neural-related genes, it remains unknown whether RE1 can be regulated to achieve trans-differentiation from glial cells to neurons. In this study, through genetic engineering of the endogenous RE1-binding protein, the zinc finger domain (ZFD) of the endogenous protein REST, which can bind to RE1, is used to regulate RE1 and realize its regulation of the related genes expression. In previous studies, fibroblasts were trans differentiated into Tuj-1 and Map2 positive neurons, but not mature neurons, by overexpressing the REST zinc finger domain or knocking down REST expression in cultured cells. Moreover, these studies were carried out in cells cultured in vitro, and most of the functions achieved in vitro are difficult to achieve in more complex in vivo systems. Moreover, in previous studies, only ordinary neurons were obtained through trans-differentiation, the special types of neurons, such as dopamine neurons, serotonin neurons, retinal ganglion cells and photoreceptor cells, could not be obtained. Therefore, studying whether regulating RE1 can transdifferentiate glial cells into special neurons in vivo possess important scientific significance.

On the other hand, the inventors of the present application found that the N-terminal and C-terminal of the REST protein can recruit various epigenetic regulatory elements, and then negatively regulate the genes they act on. When the DNA-binding protein is combined or fused with the N-terminal and/or C-terminal of the REST protein, negative regulation can be achieved on the target gene region where the DNA-binding protein binds to.

### Trans-differentiation method

In one aspect, the present application provides a method of non-neuronal cells into functional neurons in an individual.

In another aspect, the present application provides a method of preventing and/or treating a disease associated with neuronal dysfunction or death in an individual in need thereof, the method comprises transdifferentiating the non- neuronal cells into functional neurons at the position affected by the disease.

### i) Functional neurons

In the present application, the term "functional neurons" refer to the neuron cells capable of specific functions, such as dopamine neurons, retinal ganglion cells, photoreceptor cells and other neurons with specific functions. In some embodiments, the functional neurons have at least one morphological characteristic of a neuron, e.g., has synapses, e.g., axons. In some embodiments, the functional neurons express at least one marker of a mature neuron, such as a NeuN gene expression product. In some embodiments, the functional neurons have electrophysiological properties.

Functional neurons can have different functions. In some embodiments, the functional neurons include dopamine neurons, retinal ganglion cells, photoreceptor cells and cochlear spiral ganglion cells, GABA neurons, 5-HT neurons, glutamatergic neurons, ChAT neurons, NE neurons, motor neurons, spinal cord neurons, spinal motor neurons, spinal cord sensory neurons, bipolar cells, horizontal cells, amacrine cells, pyramidal neurons, interneurons, medium spiny neurons (MSN), Purkinje cells, granule cells, olfactory sensory neurons, periglobular cells, or any combination thereof.

In some embodiments, the functional neurons express the NeuN gene. The NeuN gene is a known specific marker of mature neurons. Detection of NeuN gene expression products (such as NeuN protein) in non-neuronal cells suggests that non-neuronal cells have transdifferentiated into functional neurons.

In some embodiments, the functional neurons have an axon. The axon of neurons can be observed through a microscope.

In some embodiments, the functional neurons comprise dopamine neurons, retinal ganglion cells, photoreceptor cells, or cochlear spiral ganglion cells.

In some embodiments, the functional neurons comprise dopamine neurons. In the present application, dopamine neurons and dopaminergic neurons are used interchangeably. Dopaminergic neurons are neurons that contain and release dopamine (DA) as a neurotransmitter. Dopaminergic neurons are the major source of dopamine in the central nervous system. Dopamine belongs to catecholamine neurotransmitters, which can affect neural functions such as emotion and reward, and play an important biological role in the central nervous system. The dopaminergic neurons in the brain are mainly concentrated in the substantia nigra pars compacta (SNc), ventral tegmental area (VTA), hypothalamus and periventricular areas of the midbrain. Many experiments have confirmed that dopaminergic neurons are closely related to various diseases of the human body, the most typical being Parkinson's disease. The gradual loss of dopaminergic neurons triggers many of the motor symptoms associated with Parkinson's.

In some embodiments, the dopamine neuron expresses one or more markers are selected from tyrosine hydroxylase (TH), FoxA2, Nurr1, Pitx3, Vmat2, and DAT. A "marker" in the present application may refer to an expression product of a gene, such as mRNA or protein. Detection of the expression of one or more of these markers in functional neurons indicates that the functional neurons are dopamine neurons. Exemplary gene sequences and protein sequences of these markers are well known in the art, and can be inquired through public databases (such as the gene database and protein database of the National Center for Bioinformatics (NCBI) under the National Institutes of Health), in the present application, they are listed in Table A. Tyrosine hydroxylase (TH) is an enzyme responsible for catalyzing the conversion of the amino acid L-tyrosine into dihydroxyphenylalanine (dopa), which is involved in dopamine anabolism in dopaminergic neurons. Other markers of dopamine neurons include FoxA2, Nurr1, Vmat2 and DAT, etc. In some embodiments, the dopamine neurons express NeuN, TH, and DAT.

In some embodiments, the functional neurons comprise retinal ganglion cells. Retinal ganglion cells are a type of neuron located near the inner surface of the retina (the ganglion cell layer) that receive visual information from photoreceptors through two types of interneurons (bipolar cells and amacrine cells). Its dendrites mainly establish synaptic connections with bipolar cells, and its axons extend to the optic nerve head, forming the optic nerve, which extends to the brain.

In some embodiments, the retinal ganglion cells (RGCs) express one or more markers selected from RBPMS, Pax6, Brn3a, Brn3b, Bm3c, and Map2. RBPMS is a specific marker of RGCs. If the expression of RBPMS is detected in functional neurons cells, it is suggested that the functional neurons are RGCs. Exemplary gene sequences and protein sequences of RBPMS are well known in the art, can be queried through public databases, and are listed in Table A in the present application. In some embodiments, the retinal ganglion cells (RGCs) express NeuN and RBPMS.

In some embodiments, the functional neurons comprise photoreceptor cells. Photoreceptor cells are specialized neuroepithelial cells found in the retina that have the function of sensing light and performing phototransduction functions. It can be processed by bipolar cells and ganglion cells to convert light signals into electrical signals and transmit them to the brain. Photoreceptor cells include rods and cones.

In some embodiments, the photoreceptor cells express one or more markers selected from Rhodopsin, mCAR, m-opsin and S-opsin. Rhodopsin, mCAR, m-opsin, and S-opsin are all specific markers of RGCs. Detecting the expression of Rhodopsin, mCAR, m-opsi and/or S-opsin in functional neurons indicates that the functional neurons are photoreceptor cells. Exemplary gene sequences and protein sequences of Rhodopsin, mCAR, m-opsi and S-opsin are well known in the art, which can be inquired through public databases. They are listed in Table A in the present application. In some embodiments, the photoreceptor cells express NeuN, Rhodopsin and/or mCAR.

In some embodiments, the functional neurons comprise cochlear spiral ganglion cells. The cochlear spiral ganglion cells are a kind of bipolar ganglion cells, which are the first-order neuron of the auditory conduction pathway. Their peripheral processes are connected with hair cells, and the central processes participate in the formation of the auditory nerve. Spiral ganglion cells play an important role in the transmission and encoding of sound signals.

In some embodiments, the cochlear spiral ganglion cells express one or more markers selected from NeuN, Prox1, Tuj-1, and Map2. The expression of Prox1 and Map2 detected in functional neurons indicates that the functional neurons are cochlear spiral ganglion cells. Exemplary gene sequences and protein sequences of Prox1, Tuj-1, and Map2 are well known in the art, which can be queried through public databases. They are listed in Table A in the present application. In some embodiments, the cochlear spiral ganglion cells express NeuN, Prox1, Tuj-1, and Map2.

**Table 1 Markers of the functional neurons**

| | GeneID (mouse) | Protein ID NCBI reference No. (mouse) | GeneID (human) | Protein ID NCBI reference No. (human) |
|---|---|---|---|---|
| NeuN | 52897 | NP_001034256.1 | 146713 | NP_001337382.1 |
| TH | 21823 | NP_033403.1 | 7054 | NP_954986.2 |
| FOXA2 | 15376 | NP_001277994.1 | 3170 | NP_068556.2 |
| NURR1 | 18227 | NP_038641.1 | 4929 | NP_006177.1 |
| Pitx3 | 18742 | NP_032878.1 | 5309 | NP_005020.1 |
| VMAT2 | 214084 | NP_766111.1 | 6571 | NP_003045.2 |
| DAT | 13162 | NP_034150.1 | 6531 | NP_001035.1 |
| RBPMS | 19663 | NP_001036139.1 | 11030 | NP_001008710.1 |
| Pax6 | 18508 | NP_001231127.1 | 5080 | NP_000271.1 |
| Brn3a | 18996 | NP_035273.3 | 5457 | NP_006228.3 |
| Brn3b | 18997 | NP_620394.2 | 5458 | NP_004566.2 |
| Brn3c | 18998 | NP_620395.2 | 5459 | NP_002691.1 |
| Rhodopsin | 212541 | NP_663358.1 | 6010 | NP_000530.1 |
| MCAR | 170735 | NP_573468.1 | 407 | NP_004303.2 |
| m-opsin | 14539 | NP_032132.1 | 2652 | NP_000504.1 |
| S-opsin | 12057 | NP_031564.1 | 611 | NP_001372054.1 |
| PROX1 | 19130 | NP_032963.1 | 5629 | NP_001257545.1 |
| TUJ1 | 22152 | NP_075768.1 | 10381 | NP_006077.2 |
| MAP2 | 17756 | NP_001035023.1 | 4133 | NP_001362434.1 |

### ii) Non-neuronal cells

In some embodiments, the non-neuronal cells comprise glial cells (e.g., neuroglia cells), fibroblasts, stem cells, neural precursor cells, or neural stem cells. In some embodiments, the non-neuronal cells include glial cells (e.g., neuroglia cells).

In some embodiments, the glial cells are selected from astrocytes, oligodendrocytes, ependymal cells, Schwann cells, NG2 cells, satellite cells, Müller glia cells, inner ear Glial cells and any combination thereof. Müller glia cells (MG) are the main glial cell in retinal tissue.

In some embodiments, the glial cells are located in the brain, spinal cord, eye or ear. In some embodiments, the glial cells are located in the striatum, substantia nigra, ventral tegmental area of the midbrain, medulla oblongata, hypothalamus, dorsal midbrain, or cerebral cortex of the brain.

In some embodiments, the active substance is administered locally to glial cells in one or more of the following locations in the individual: 1) glial cells in the striatum; ii) in the substantia nigra of the brain iii) glial cells in the retina; iv) glial cells in the inner ear; v) glial cells in the spinal cord; vi) glial cells in the prefrontal cortex; vii) glial cells in the motor cortex cells; viii) glial cells in the hypothalamus; and ix) glial cells in the ventral tegmental area (VTA).

Without wishing to be bound by any theory, it is believed that the microenvironment of glial cells in vivo helps to promote the trans-differentiation induced by the active substances described in the present application, and promotes the trans-differentiation of glial cells in vivo into functional neurons.

In some embodiments, the glial cells are selected from astrocytes, Müller glial cells, and cochlear glial cells.

In some embodiments, the glial cells comprise astrocytes, and the functional neurons comprise dopamine neurons. In some embodiments, the methods provided herein relate to methods of transdifferentiating astrocytes into dopamine neurons in an individual. In some embodiments, the astrocytes are located in the striatum and/or the substantia nigra. In some embodiments, the method comprises administering an active substance provided herein to the individual's striatum and/or substantia nigra.

In some embodiments, the glial cells comprise Müller glial cells, and the functional neurons comprise retinal ganglion cells (RGCs) and/or photoreceptor cells. In some embodiments, the methods provided herein relate to methods of transdifferentiating Müller glial cells into retinal ganglion cells (RGCs) and/or photoreceptor cells in an individual. In some embodiments, the Müller glial cells are located in the retina or vitreous cavity. In some embodiments, the method comprises administering an active substance provided herein to the subretinal or vitreous cavity of the individual.

In some embodiments, the glial cells comprise cochlear glial cells and the functional neurons comprise cochlear spiral ganglion cells. In some embodiments, the methods provided herein relate to methods of transdifferentiating cochlear glial cells into cochlear spiral ganglion cells in an individual. In some embodiments, the cochlear glial cells are located in the inner ear. In some embodiments, the method comprises administering an active agent provided herein to the inner ear of the individual.

In some embodiments, the trans-differentiation efficiency of the glial cells into functional neurons achieved after the administration of the active substance is at least 1%, or at least 10%, 20%, 30%, 40% % or 50%. Trans-differentiation efficiency can be detected and calculated by methods known to the skilled persons in the art. For example, fluorescent proteins can be used to label the initial cells of trans-differentiation (such as glial cells), such as GFAP-mCherry, GFAP-tdTomato, GFAP-EGFP, etc. It is also possible to use, for example, Ai9 transgenic mice in which the glial cells have fluorescent labeling. Since the transdifferentiated cells also carry fluorescence, the trans-differentiation efficiency can be calculated by calculating the percentage of the number of transdifferentiated cells to the number of initially labeled cells. Alternatively, the trans-differentiation efficiency can also be calculated as a percentage of the number of cells produced by trans-differentiation compared to the number of cells of this type at the site of administration, for example, in the substantia nigra, the percentage of newly generated dopamine neurons to dopamine neurons in the substantia nigra.

### iii) Active substances

In some embodiments, the method for transdifferentiating non-neuronal cells into functional neurons in an individual provided in the present application includes administering to the individual an agent that can reduce the binding of REST to RE1/NRSE elements, or reduce the amount of REST or active active substance.

In some other embodiments, the method for preventing and/or treating diseases associated with neuronal dysfunction or death in an individual in need provided in the present application includes administration of a therapeutically effective dose of an active substance capable of reducing the binding of REST to the RE1/NRSE element, or reducing the amount or activity of REST, transdifferentiates non-neuronal cells into functional neurons at the site affected by the disease.

In some embodiments, the active substance is capable of reducing the amount or activity of REST. Any active substance that reduces the amount or activity of REST can be used. In some embodiments, the amount of REST is reduced by methods such as gene editing, small RNA interference, or accelerated protein degradation. In some embodiments, the amount of REST is reduced by methods such as gene editing, antisense oligonucleotide (Antisense Oligonucleotide, ASO), small RNA interference, miRNA technology, small molecule compounds, or accelerated protein degradation. In some embodiments, the REST inhibitory active region is removed by gene editing or the REST activity is reduced by an inhibitor of REST activity.

In some embodiments, the active agent is capable of reducing REST binding to the RE1/NRSE element. In some embodiments, the binding of a REST-binding agent to REST could block the binding of REST to the REINRSE element. One example of REST-binding agent is a REST antibody.

In some embodiments, the active substance includes a RE1/NRSE element blocker, which can bind to the RE1/NRSE element to block the binding of REST and the RE1/NRSE element.

In some embodiments, the RE1/NRSE element blocker comprises a small molecule compound, nucleic acid, or nucleic acid analog that competes with REST for binding to RE1.

In some embodiments, the RE1/NRSE element blocker comprises a protein or a nucleic acid encoding the protein that competes with REST for binding to RE1.

In some embodiments, the protein that competes with REST for binding to RE1 comprises a REST variant. "Variant" in the present application refers to a derivative sequence having one or more substitutions (including but not limited to conservative substitutions), additions, deletions, insertions or truncations, or any combination thereof, compared with the parent sequence. In certain embodiments, a REST variant may comprise a fragment of REST protein, or a fusion protein of a fragment of a REST protein with another protein.

In some embodiments, the REST variant comprises the DNA binding domain of REST but lacks the N-terminal and/or C-terminal repression domain of REST. Native REST proteins contain N-terminal repression domain, DNA-binding domain responsible for binding to DNA at the middle position, and C-terminal transcription repression domain. There are eight zinc finger domains in the human REST protein, which are responsible for binding to DNA, and more specifically binding to RE1/NRSE elements. The DNA binding domain of REST can be the eight zinc finger domains in the REST protein or the fragments thereof capable of binding to DNA (for example, zinc finger domains with less than eight zinc finger domains).

In some embodiments, the REST variant comprises amino acids from 155 to 420 of REST (especially human REST), but lacks the N-terminal and/or C-terminal inhibitory domain of REST. In some embodiments, the REST variant comprises a RE1-binding fragment in the amino acids from positions 155 to 420 of REST (especially human REST), but lacks the N-terminal and/or C-terminal inhibitory domain of REST. "RE1-binding fragment" refers to a protein fragment capable of binding RE1 element in the present application.

In some embodiments, the REST variant comprises the amino acid sequence of SEQ ID NO: 1, 3, 5 or 9, or a sequence having at least 70%, 60%, or 50% identity percentage with anyone thereof.

In some embodiments, the RE1/NRSE element blocker comprises the nucleic acid encoding the REST variant, the nucleic acid encoding the REST variant has SEQ ID NO: 2, 4, 6 or 10, or a sequence having at least 70%, 60%, or 50% identity percentage with anyone thereof.

The term "sequence identity percentage (%)" with respect to amino acid sequences (or nucleic acid sequences) is defined as after aligning the sequences and introducing gaps when necessary to achieve the maximum number of identical amino acids (or nucleotides), the percentage of amino acid (or nucleotide) residues in the candidate sequence that are identical to those in the reference sequence is calculated. In other words, the sequence identity percentage (%) of an amino acid sequence (or a nucleic acid sequence) can be calculated by dividing the number of identical amino acid residues (or bases) with respect to the reference sequence to the number of amino acid residues (or bases) in the candidate sequence or reference sequence (taking the shorter one as the basis). Conservative substitutions of amino acid residues may or may not be considered identical residues. Alignment for the purpose of determining percent amino acid (or nucleic acid) sequence identity can be achieved, for example, using publicly available tools such as BLASTN, BLASTp (found at the U.S. National Center for Biotechnology Information ; NCBI), see also Altschul S.F. et al., J. Molecular Biology, 215:403-410 (1990); Stephen F. et al., Nucleic Acids Res., 25:3389- 3402 (1997), ClustalW2 (available at the European Bioinformatics Institute website), see also Higgins D.G. et al., Methods in Enzymology, 266:383-402 (1996); Larkin M.A. et al., Bioinformatics (Oxford, England), 23(21): 2947-8 (2007)) and ALIGN or Megalign (DNASTAR) software. Those skilled persons in the art can use the default parameters provided by the tool, or customize the parameters of the alignment if desired, e.g., by selecting a suitable algorithm.

In some embodiments, the nucleic acid encoding the REST variant is codon-optimized, optionally the nucleic acid comprises the nucleotide sequence shown in SEQ ID NO: 15, or comprises the nucleotide sequence having at least 70%, 60%, or 50% sequence identity percentage of SEQ ID NO: 15. Without being bound by theory, it is believed that the nucleic acid sequence of the codon-optimized REST variant is capable of higher expression in human cells.

In some embodiments, the REST variant further comprises an activation domain fused to the REST DNA binding domain. In the present application, "activation domain" refers to a domain capable of interacting with the regulatory sequence of a target gene and activating or increasing the expression of the target gene.

In some embodiments, the activation domain comprises epigenetic modification proteins or gene activation regulatory elements, optionally, the activation domain comprises VP64, P65-HSF1, VP16, RTA, Suntag, P300, CBP or any combination thereof, optionally, the activation domain includes VP64 or P65-HSF1.

In some embodiments, the REST variant is fused to one or more nuclear localization signal sequences (NLS). Without wishing to be bound by any theory, it is believed that the nuclear localization signal sequence can promote the REST variant to enter the nucleus, thereby better regulating gene expression and promoting cell trans-differentiation. Any suitable nuclear localization signal sequence can be used. Examples of nuclear localization signal sequences include, but are not limited to, BPNLS (e.g., the amino acid sequence shown in SEQ ID NO: 13), the NLS of the SV40 viral large T antigen, having the amino acid sequence as PKKKRKV (SEQ ID NO: 41); the NLS of nucleoplasmin (e.g., a NLS of bipartite nucleoplasmin having a sequence as KRPAATKKAGQAKKKK) (SEQ ID NO:42); a NLS of c-myc having the amino acid sequence as PAAKRVKLD (SEQ ID NO:43) or RQRRNELKRSP (SEQ ID NO:44 ); a NLS of hRNPA1M9 having a sequence as NQSSNFGPMKGGNFGGRSSGPYGGGGQYFAKPRNQGGY (SEQ ID NO: 45); a sequence RMRIZFKNKGKDTAELRRRRVEVSVELRKAKKDEQILKRRNV (SEQ ID NO: 46) from the IBB domain of importin-alpha; a sequence VSRKRPRP (SEQ ID NO: 47) and PPKKARED (SEQ ID NO:48) from myoma T protein; a sequence POPKKKPL (SEQ ID NO:49) from human p53; a sequence SALIKKKKKMAP (SEQ ID NO:50) from mouse c-abl IV; a sequence DRLRR (SEQ ID NO:51) and PKQKKRK (SEQ ID NO:52)of influenza virus NS1; a sequence RKLKKKIKKL (SEQ ID NO:53) from hepatitis delta antigen; a sequence REKKKFLKRR (SEQ ID NO:54) from mouse Mx1 protein; a sequence KRKGDEVDGVDEVAKKKSKK (SEQ ID NO:55) from human poly(ADP-ribose) polymerase; and a sequence RKCLQAGMNLEARKTKK (SEQ ID NO:56) from the steroid hormone receptor (human) glucocorticoid. Non-limiting examples of NES include the NES sequences LYPERLRRILT (SEQ ID NO: 57), PKKKRKV (SEQ ID NO: 58).

In some embodiments, at least one of said nuclear localization signal sequences is fused to the N-terminus of said REST variant.

In some embodiments, at least one of said nuclear localization signal sequences is fused to the C-terminus of said REST variant.

In some embodiments, at least one of said nuclear localization signal sequences is fused to the N-terminus and C-terminus of said REST variant, respectively.

In some embodiments, the nuclear localization signal sequence comprises an amino acid sequence selected from: SEQ ID NOs: 13, and 41-58.

In the examples of the present application, the inventors used protein structure prediction, combined with genetic engineering technology, to conduct a series of analyzes and modifications to the endogenous zinc finger domain of REST. First, through truncation experiments, the inventors found that overexpressing the amino acids 155-420 of REST (which only contains 8 zinc finger domains, called RZFD: REST Zinc Finger Domain), can block the binding of REST to RE1. Overexpression of RZFD in mouse striatal astrocytes can transdifferentiate glial cells into functional neurons by AAV-mediated gene delivery technology. Through further modification of RZFD, VP64 was fused to the N-terminal or C-terminal of RZFD to form RZFD-VP64, and AAV-mediated trans-differentiation from glial cells to neurons in vivo was successfully achieved. The transcriptional activation domains of P65 and HSF1 were further fused on RZFD to form RZFD-P65-HSF1. Using AAV-mediated in vivo trans-differentiation technology, we found that RZFD-P65-HSF1 can also transdifferentiate glial cells into functional neurons in the striatum.

Previous studies have shown that inhibiting REST can transdifferentiate glial cells into neurons, and the REST binds to a DNA sequence in the genome called RE1. RE1 have a class of sequences so that cannot be targeted through the CRISPR technology. In this study, a human endogenous zinc finger structure (REST zinc finger domain (RZFD)) was used cleverly to target the RE1 sequence, thereby blocking the silencing complex REST binding to RE1. Expressing RZFD (RZFD-V1) in non-neuronal cells such as glia cell relieves the inhibition of REST silencing complex on neuron-associated gene. Further, by fusing activation domains such as VP64 (RZFD-V2) or P65-HSF1 (RZFD-V3) on RZFD, the expression of neuron-related genes was further promoted, and the trans-differentiation of glial cells into neurons was promoted. In the examples of the present application, it was also found that RZFD-V1, RZFD-V2 and RZFD-V3 can transdifferentiate glial cells into dopamine neurons through immunofluorescence staining and DAT-Cre: Ai9 labeling system.

In the example of the present application, by injecting AAV into Ai9 retina, it was found that RZFD, RZFD-VP64 and RZFD-P65-HSF1 could transdifferentiate Müller cells into retinal ganglion cells and some photoreceptor cells were also observed. Retinal ganglion cells are the only cells in the visual pathway that carry visual signals to the brain, and their absence or death can lead to permanent blindness. Similar results have been found in non-human primate studies as compared to those in mice, the inventors found that RZFD, RZFD-VP64 and RZFD-P65-HSF1 can transdifferentiate glial cells into dopamine neurons in the brain, and RZFD, RZFD-VP64 and RZFD-P65-HSF1 can transdifferentiate Müller cells into retinal ganglion cells and photoreceptor cells in the retina.

### iv) Individual and disease treatment

In some embodiments, the individual is a human or an animal.

In some embodiments, the animal is a non-human primate (e.g., monkey), rat or mouse.

In the context of the present application, diseases associated with neuronal dysfunction or death mainly include diseases associated with dysfunction or death of dopamine neurons, and visual impairment related to loss or death of optic ganglion or photoreceptor cells. In some embodiments, the disease associated with neuronal dysfunction or death is selected from: Parkinson's disease, Alzheimer's disease, stroke, schizophrenia, Huntington's disease, depression, motor neuron disease, amyotrophic lateral sclerosis, spinal muscular atrophy, Pick disease, sleep disorders, epilepsy, ataxia, visual impairment due to RGC cell death, glaucoma, age-related RGC lesions, optic nerve damage, retinal ischemia or hemorrhage, Leber hereditary optic neuropathy, degeneration or death of photoreceptor cells due to damage or degeneration, macular degeneration, retinitis pigmentosa, diabetic-related blindness, night blindness, color blindness, hereditary blindness, amaurosis congenita, deafness or hearing loss due to spiral ganglion cell death, and any combination thereof.

In some embodiments, the application provides a method for preventing and/or treating diseases associated with neuronal dysfunction or death in an individual in need thereof, comprising administering a therapeutically effective dose of the active substance provided in this application into the striatum and/or substantia nigra of the individual, in order to transdifferentiate the astrocytes in the striatum and/or substantia nigra into dopamine neurons, wherein the disease associated with neuronal dysfunction or death is selected from: Parkinson's disease, depression and Alzheimer's disease.

In some embodiments, the application provides a method for preventing and/or treating diseases associated with neuronal dysfunction or death in an individual in need thereof, comprising administering a therapeutically effective dose of the active substance provided in this application into the subretinal or vitreous cavity of the individual, in order to transdifferentiate the Müller glial cells in the retina or vitreous cavity into retinal ganglion cells (RGC) and/or photoreceptor cells, wherein the diseases associated with neuronal dysfunction or death are selected from: vision impairment due to RGC cell death, glaucoma, age-related RGC lesions, optic nerve damage, retinal ischemia or hemorrhage, Leber hereditary optic neuropathy, degeneration or death of photoreceptor cells due to damage or degeneration, macular degeneration, retinitis pigmentosa, diabetic-related blindness, night blindness, color blindness, hereditary blindness, and amaurosis.

In some embodiments, the present application provides a method for preventing and/or treating diseases associated with neuronal dysfunction or death in an individual in need thereof, comprising administering a therapeutically effective dose of the active substance provided in this application to the inner ear of the individual, in order to transdifferentiate the cochlear glial cells in the inner ear into cochlear spiral ganglion cells, wherein the disease associated with neuronal dysfunction or death is selected from: deafness or hearing decrease caused by death of spiral ganglion cells.

### REST variant

In another aspect, the present application provides a REST variant comprising the DNA binding domain of REST but lacking the N-terminal and/or C-terminal repression domain of REST. In the present application, REST refers to native or endogenous REST molecules, including REST from any species.

In some embodiments, the REST variant contains amino acids from 155 to 420 of REST, but lacks the N-terminal and/or C-terminal inhibitory domain of REST. In some embodiments, the REST variant comprises a RE1-binding fragment in the amino acids 155--420 of REST (especially human REST), but lacks the N-terminal and/or C-terminal inhibitory domain of REST.

In some embodiments, the REST variant comprises the amino acid sequence of SEQ ID NO: 1, 3, 5 or 9, or a sequence having at least 70%, 60%, or 50% identity percentage with anyone thereof.

In some embodiments, the RE1/NRSE element blocker comprises nucleic acid encoding the REST variant, the nucleic acid encoding the REST variant has SEQ ID NO: 2, 4, 6 or 10 , or a sequence having at least 70%, 60%, or 50% identity percentage with anyone thereof.

In some embodiments, the REST variant further comprises an activation domain fused to the REST DNA binding domain.

In some embodiments, the activation domain comprises an epigenetic modification protein or a gene activation regulatory element, optionally, the activation domain comprises VP64, P65-HSF1, VP16, RTA, Suntag, P300, CBP or any combination thereof, optionally, the activation domain comprises VP64 or P65-HSF1.

In some embodiments, the REST variant is fused to one or more nuclear localization signal sequences.

In some embodiments, at least one of said nuclear localization signal sequences is fused to the N-terminus of said REST variant.

In some embodiments, at least one of said nuclear localization signal sequences is fused to the C-terminus of said REST variant.

In some embodiments, at least one of said nuclear localization signal sequences is fused to the N-terminus and C-terminus of said REST variant, respectively.

In some embodiments, the nuclear localization signal sequence comprises the amino acid sequence shown in SEQ ID NO: 13.

### Polynucleotides encoding REST variants

In another aspect, the application provides a polynucleotide comprising a nucleic acid sequence encoding a REST variant as described in the application.

In another aspect, the application provides an expression vector, which comprises a polynucleotide encoding a REST variant, and optionally further comprises a promoter operably linked to the polynucleotide.

In some embodiments, the promoter is a glial cell-specific promoter. In some embodiments, the glial cell-specific promoter is an astrocyte-specific promoter or a Müller glia (MG) cell-specific promoter.

In some embodiments, the glial cell-specific promoter is selected from GFAP promoter, ALDH1L1 promoter, EAAT1/GLAST promoter, glutamine synthetase promoter, S100β promoter EAAT2/GLT-1 promoter and the Rlbp1 promoter, preferably selected from the GFAP promoter.

In some embodiments, the glial cell-specific promoter is a cochlear glial cell-specific promoter.

In some embodiments, the cochlear glial cell-specific promoter is selected from: GFAP promoter (for example, see SEQ ID NO: 39 or 40), ALDH1L1 promoter, EAAT1/GLAST promoter, and Plp1 promoter.

### Pharmaceutical composition, medicine box, kit

In another aspect, the application provides a pharmaceutical composition comprising the REST variant as described in the application, or a polynucleotide encoding the REST variant, or a expression vector comprising a polynucleotide encoding the REST variant, and a pharmaceutically acceptable carrier.

In some embodiments, the pharmaceutical composition further comprises a carrier for delivering the polynucleotide, wherein the carrier comprises a viral vector, liposome, nanoparticle, exosome, or virus-like particles.

In some embodiments, the viral vectors include recombinant adeno-associated viral vectors (rAAV), adeno-associated viral (AAV) vectors, adenoviral vectors, lentiviral vectors, retroviral vectors, poxvirus vectors, herpes virus vectors, SV40 viral vectors, or any combination thereof, wherein AAV or rAAV is preferred.

In some embodiments, the pharmaceutical composition is suitable to administrated locally to glial cells in one or more of the following sites: 1) glial cells in the striatum; ii) glial cells in the substantia nigra of the brain iii) glial cells in the retina; iv) glial cells in the inner ear; v) glial cells in the spinal cord; vi) glial cells in the prefrontal cortex; vii) glial cells in the motor cortex; viii) glial cells in the hypothalamus; and ix) glial cells in the ventral tegmental area (VTA).

In some embodiments, the pharmaceutical composition is suitable for intracranial or intraocular administration.

In some embodiments, the pharmaceutical composition further comprises i) one or more dopamine neuron-associated factors, or ii) one or more retinal ganglion cell-related factors which expressing in Müller glial cells,
1) the one or more dopamine neuron-related factors are selected from: FoxA2, Lmx1a, Lmx1b, Nurr1, Pbxla, Pitx3, Gata2, Gata3, FGF8, BMP, En1, En2, PET1, Pax family proteins (Pax3, Pax6, etc.), SHH, Wnt family proteins, TGF-β family proteins, and any combination thereof;
2) the one or more retinal ganglion cell-related factors are selected from: β-catenin, Oct4, Sox2, Klf4, Crx, aCamKII, Brn3a, Brn3b, Brn3C, Math5, Otx2, Ngn2, Ngn1, AscL1, miRNA9, miRNA-124, Nr2e3, Nrl, and any combination thereof.

In another aspect, the application provides a medicine box or kit comprising the pharmaceutical composition provided in the application.

### Fusion proteins and protein complexes

In another aspect, the application provides a fusion protein comprising a DNA binding protein fused to one or more REST inhibitory domains. The DNA binding protein can bind to the target DNA to be regulated, and allow the fused REST inhibitory domain to negatively regulate the target DNA to be regulated, so as to inhibit the transcriptional activity of the target DNA to be regulated.

In addition to direct fusion, DNA-binding proteins and one or more REST inhibitory domains can also be combined or complexed non-covalently to form protein complexes.

Therefore, the application also provides a composition comprising a) a DNA binding protein and b) a protein comprising one or more REST inhibitory domains, wherein said a) and b) can be combined to form a protein complex. In the present application, "protein complex" refers to a complex formed by two protein molecules bound by non-covalent bonds. For example, the DNA-binding protein may additionally have a binding moiety (such as an antibody or an antigen-binding fragment thereof) capable of binding to the protein comprising one or more REST inhibitory domains. In another example, a protein comprising one or more REST inhibitory domains may additionally have a binding moiety (e.g., an antibody or an antigen-binding fragment thereof) capable of binding the DNA-binding protein. Those skilled persons in the art can understand that the binding part can neither affect the binding of the DNA-binding protein to the target DNA, nor the binding of the protein comprising one or more REST inhibitory domains to RE1. In other words, the formed protein complex still has at least part of the functions of binding the target DNA and at least part of the functions of binding RE1. In this way, a non-covalently bound protein complex can be obtained, which contains both the DNA binding protein, which can bind the target DNA to be regulated, and the inhibitory domain of REST, which can inhibit the target DNA to be regulated transcriptional activity.

In some embodiments, in the compositions provided by the present application, the a) and b) are respectively connected with a pair of self-assembled assemblies, and the self-assembled assemblies can be combined with each other. In the present application, "self-assembling assembly" refers to two ligands that can spontaneously bind to each other. Since the two ligands are capable of spontaneous association, they are also called a pair of self-assembling assemblies.

In some embodiments, the pair of self-assembling assemblies can be selected from: i) two protein domains which could be integrated with each other; and ii) an RNA splice donor and a splice acceptor. Any two protein domains that can bind to each other can be used as the self-assembling assembly described in the present application, for example: antigen and antibody; antigen-binding fragments in antigen and antibody; receptor and ligand; or the two proteins which could be integrated with each other. In some embodiments, the pair of self-assembling assemblies includes: KFBP and FRB; or PYL and ABI. Any RNA splicing donor and splicing acceptor can be used as the self-assembly assembly described in the present application, for example, a split intein can be used.

In some embodiments, the two ligands of the pair of self-assembling assemblies are respectively linked to: a) a DNA binding protein and b) a protein comprising one or more REST repression domains. For example, the inhibitory domain of REST can be linked to an antigen, and the CRISPR-Cas protein can be linked to an antibody bound to the antigen, thereby the protein complex described in the present application could be self-assembled through the interaction between the antigen and the antibody.

### i) Inhibition domain of REST

The fusion proteins, compositions and protein complexes provided in the present application all have one or more REST inhibitory domains. In the present application, the "REST inhibitory domain" refers to a domain in the REST protein that has the function of inhibiting gene expression. Without wishing to be bound by any theory, it is believed that there are different REST inhibitory domains in REST proteins, including but not limited to, the N-terminal region of the REST protein and the C-terminal region of the REST protein. In the present application, the "one or more REST inhibitory domains" may refer to one or more same REST inhibitory domains, such as multiple repetitions of the same inhibitory domain, or one or more different REST inhibitory domains.

In some embodiments, the one or more REST inhibitory domains comprise an N-terminal inhibitory domain of REST and/or a C-terminal inhibitory domain of REST.

In some embodiments, the one or more REST inhibitory domains are derived from human REST protein or animal REST protein. In some embodiments, the animal REST protein may include non-human primate REST protein (such as monkey), rodent REST protein (such as mouse, rat), poultry (such as chicken, duck, goose etc.), farm animals (e.g. cattle, sheep, pigs, etc.).

In some embodiments, the N-terminal inhibitory region of REST comprises amino acids from positions 1 to 83 of REST or a fragment thereof having transcriptional inhibitory activity. In the present application, "transcriptional repression activity" means that when the repression domain of REST interacts with the transcriptional regulatory sequence of a target gene, the repression domain of REST can reduce the transcription of the target gene. In the present application, "a fragment having transcriptional repressive activity" refers to a fragment capable of providing at least part of the transcriptional repressive activity in the amino acid sequence of REST.

In some embodiments, the N-terminal inhibitory region of the REST comprises the sequence shown in SEQ ID NO: 16 or a fragment thereof having transcriptional inhibitory activity.

In some embodiments, the C-terminal inhibitory region of REST comprises amino acids from positions 1008 to1097 of REST or a fragment thereof having transcriptional inhibitory activity.

In some embodiments, the C-terminal inhibitory region of the REST comprises the sequence shown in SEQ ID NO: 18 or a fragment thereof having transcriptional inhibitory activity.

In some embodiments, the fragment with transcriptional repression activity comprises a fragment of at least 20 consecutive amino acids, 30 consecutive amino acids, 40 consecutive amino acids, or 50 consecutive amino acids of the REST protein.

### ii) DNA binding proteins

The fusion protein, composition, and protein complex provided in this application all contain DNA-binding proteins. In some embodiments, the DNA binding protein can be targeted to bind to a specific target DNA sequence. In some embodiments, the DNA-binding protein can be a transcription activator-like effector nuclease (TALEN), a zinc finger ribozyme (ZFN), a sequence-guided DNA-binding protein such as a CRISPR-Cas protein, or a DNA binding moiety of a transcription factor. ZFNs and TALENs achieve specific DNA binding through protein-DNA interactions, and CRISPR-Cas proteins (such as Cas9, Cas12) are guided by short guide RNAs that base-pair with the target DNA to bind to specific DNA sequences. In some embodiments, the DNA binding moiety of the transcription factor is not the naturally occurring DNA binding domain of REST.

In some embodiments, the DNA-binding protein is selected from sequence-guided DNA-binding protein. "Sequence-guided DNA-binding protein" refers to a DNA-binding protein that can bind to a specific nucleotide sequence under the guidance of a targeting moiety. Sequence-guided DNA-binding proteins include, but are not limited to, CRISPR-Cas proteins.

In some embodiments, the sequence-guided DNA binding protein is a CRISPR-Cas protein or a variant thereof. "CRISPR-Cas protein" refers to the protein associated with clustered regularly interspaced short palindromic repeat, which is a type of nuclease derived from the adaptive immune system of bacteria or archaea. CRISPR-Cas protein can be used to bind to and cut a specific nucleic acid sequence targeted by a guide RNA (gRNA). CRISPR-Cas proteins capable of binding to DNA include but are not limited to Cas9 and Cas12.

In some embodiments, the CRISPR-Cas protein variant does not have nuclease activity.

In some embodiments, the sequence-guided DNA binding protein is a Cas9 protein or a Cas9 variant which does not have nuclease activity. Examples of Cas9 variants that do not have nuclease activity include, nCas9 or dCas9.

Cas9 proteins can be derived from a variety of bacteria, including, but not limited to, Cas9 from Streptococcus pyogenes (SpCas9), Cas9 from Staphylococcus aureus (SaCas9), Cas9 from Streptococcus thermophilus Cas9 (StCas9), etc. In some embodiments, the Cas9 protein can be engineered to contain one or more mutations that reduce or eliminate nuclease activity. Mutations in the Cas9 protein can render it incapable of cleaving double-stranded DNA, or confer it the ability to cleave only a single-stranded DNA. For example, taking SpCas9 as an example, the Asp residue at position 10 can be changed to Ala residue (i.e., D10A mutant), or the His residue at position 840 can be changed to Ala residue (H840A mutant), thus the mutant that can only cut a single-stranded DNA was obtained, which is also called as nCas9. For another example, taking SpCas9 as an example, D10A and H840A double mutations can be introduced at the same time, so as to the Cas9 loses the activity of cutting DNA double strands. Such a Cas9 mutant is also called as dCas9. In some embodiments, the dCas9 has an amino acid sequence as shown in SEQ ID NO: 21 or 22.

In some embodiments, wherein the sequence-guided DNA-binding protein is a Cas12 protein or a Cas12 variant which does not have nuclease activity.

Cas12 protein, also known as Cpf1, can be derived from various bacteria, including but not limited to Cpf1of Lachnospiraceae bacterium (LbCpf1), Cpf1 of Acidaminococcussp (AsCpf1), Cpf1 of Francisella novicida (FnCpf1) etc. In some embodiments, the Cas12 protein can be engineered to contain one or more mutations that can reduce or eliminate nuclease activity.

In some embodiments, at least one of the N-terminal inhibitory domains of the REST protein is linked to the N-terminus or C-terminus of the DNA-binding protein.

In some embodiments, at least one of the C-terminal inhibitory regions of the REST protein is linked to the N- or C-terminus of the DNA-binding protein.

In some embodiments, the fusion protein comprises at least one N-terminal inhibitory domain of the REST linked in tandem to the N-terminus or C-terminus of the DNA binding protein.

In some embodiments, the fusion protein comprises at least one C-terminal inhibitory domain of the REST linked in tandem to the N-terminus or the C-terminus of the DNA binding protein.

In some embodiments, the fusion protein comprises the amino acid sequence shown in SEQ ID NO: 25-27.

### Polynucleotide

In another aspect, the application provides a polynucleotide encoding a fusion protein or composition or protein complex as described in the application.

A polynucleotide encoding a protein complex as described in the application.

In some embodiments, comprising a first polynucleotide fragment encoding a DNA binding protein and a second polynucleotide fragment encoding a protein comprising one or more REST inhibitory domains.

In some embodiments, wherein the first polynucleotide fragment and the second polynucleotide fragment are linked by a third self-cleavable nucleotide fragment.

In another aspect, the application provides a method for inhibiting the expression of a target gene in a cell, comprising delivering a fusion protein as described in the application or a polynucleotide encoding the fusion protein to the cell, wherein the DNA binding protein can bind to the target gene or its regulatory sequence, and inhibit the expression of the target gene.

In another aspect, the application provides a method for inhibiting target gene expression in an individual, comprising delivering a fusion protein as described in the application or a polynucleotide encoding the fusion protein to the individual, wherein the DNA binding protein can bind to the target gene or its regulatory sequence, and inhibit the expression of the target gene.

In some embodiments, the DNA binding protein is a CRISPR-Cas protein or a variant thereof.

In some embodiments, the CRISPR-Cas protein variant does not have nuclease activity.

In some embodiments, the sequence-guided DNA-binding protein is a Cas9 protein or a Cas9 variant that does not have nuclease activity.

In some embodiments, the method further comprises delivering a guide RNA to the cell or the individual, wherein the guide RNA comprises a targeting sequence complementary to a target region of the interest gene or its regulatory sequence, and a sequence binding to the DNA binding protein.

### EXAMPLES

### General Methods

Animal ethics:
   The feeding and use of animals in this study were conducted under the guiding principles of the Ethics Committee for Biomedical Research of the Center for Excellence in Brain Science and Intelligence Technology, Chinese Academy of Sciences.
Plasmid construction:
   In this study, all plasmids were constructed by our own laboratory. The AAV backbone vector was digested with restriction enzymes, analyzed by agarose gel electrophoresis, and then recovered. The cell cDNA was used as a template to perform PCR, and the DNA fragment was recovered by agarose gel electrophoresis. The ClonExpress MultiS One Step Cloning Kit (Vazyme, C113-02) from Nanjing vazyme Biotech Co., Ltd. was used to ligate the backbone vector with the fragment. After ligation, the plasmids were transformed into DH5a Escherichia coli and plated. On the second day, single colonies were picked for identification, and positive clones were sequenced. Clones with completely correct sequence were selected for expanded culture and plasmid extraction.
The AAV injection into the mouse brain:
   The AAV serotype used in this study was AAV8. Stereotaxic injection was performed using the RWD stereotaxic injection system in C57BL/6 or Dat-Cre: Ai9 mice aged over two months. The titers of AAV-GFAP-RZFD-V1, AAV-GFAP-RZFD-V2, and AAV-GFAP-RZFD-V3 were greater than 5×10¹² vg/ml (1-3 µl were injected per injection). The AAV was injected into the striatum (AP + 0.8 mm, ML ± 1.6 mm, and DV -2.8 mm) or the substantia nigra (AP - 3.0 mm, ML ± 1.25 mm, and DV -4.5 mm).
Immunofluorescence staining of mouse tissues:
   Two weeks and 1.5-2 months after injection, the mouse tissues were collected, sectioned, and subjected to immunofluorescence staining. After perfusion with physiological saline and 4% paraformaldehyde (PFA), the brains were removed, fixed overnight in 4% PFA, and then dehydrated in 30% sucrose for at least 12 hours until the tissues sank to the bottom of the solution. Frozen sections were proceeded after OCT embedding with a slice thickness of 30 µm or 40 µm. Before immunofluorescence staining, the brain slices were washed three times with 0.1M phosphate-buffered saline (PBS), each time for 5-10 minutes. After incubation with the primary antibody overnight at 4°C, the slices were washed 3-4 times with PBS, each time for 10-15 minutes. Subsequently, the secondary antibody diluted in antibody dilution buffer was added for incubation at room temperature for 2-3 hours. After incubation, the slices were washed again with PBS 3-4 times, each time for 10-15 minutes. Finally, the slices were mounted and preserved using anti-fade mounting medium (Life Technology).
Antibodies:
   In this study, the primary antibodies used included: guinea pig anti-NeuN (1:500, ABN90, Millipore), rabbit anti-TH (1:500, AB152, Millipore), rat anti-DAT (1:100, MAB369, Millipore), rabbit anti-RBPMS (Proteintech, Cat# 15187-1-AP), and mouse anti-Flag (1:2000, F3165, Sigma). The secondary antibodies used included: Cy5-AffiniPure Donkey Anti-Guinea Pig IgG (H+L) (1:500, 706-175-148, Jackson ImmunoResearch), Alexa Fluor-488 AffiniPure Donkey Anti-Rabbit IgG (H+L) (1:500, 711-545-152, Jackson ImmunoResearch), Alexa Fluor-488 AffiniPure Donkey Anti-Mouse IgG (H+L) (1:500, 715-545-150, Jackson ImmunoResearch), and Cy5 AffiniPure Donkey Anti-Rabbit IgG (H+L) (1:500, 711-175-152, Jackson ImmunoResearch).
Mouse model of 6-OHDA PD:
   The mice used in this experiment were adult C57BL/6 mice aged 7-10 weeks. Half an hour before anesthesia, 25 mg/kg of desipramine hydrochloride (D3900, Sigma-Aldrich) was injected intraperitoneally. After anesthesia, 3 µg of 6-OHDA (H116, Sigma-Aldrich) or physiological saline was injected into the right medial forebrain bundle of the mice at the following stereotaxic coordinates: anteroposterior (A/P) = -1.2 mm, mediolateral (M/L) = -1.1 mm, dorsoventral (D/V) = -5 mm. One hour after the surgery, 1 ml of 4% glucose-saline solution was injected subcutaneously into the mice.
Apomorphine-induced rotation test:
   Ten minutes prior to the test, mice were injected intraperitoneally with 0.5 mg/kg of apomorphine (A4393, Sigma-Aldrich). During the test, each mouse was placed in an opaque cylinder (with a diameter of 30 cm), and their behavior was recorded for 20 minutes by a camera positioned above the cylinder. Rotation was defined as a full-body turn with one hind paw serving as the pivot point and without any change in head orientation. The number of rotations towards the injection side and the contralateral side were counted and quantified as the number of contralateral rotations within the 20-minute period.
Cylinder test:
   Gently place the mouse into a glass beaker (1000ml) and record its behavior for 10 minutes using a camera positioned in front of it. Calculate the number of times the forelimb on the injection side and the contralateral side touching the wall separately, and quantify the data as the ratio of ipsilateral wall touches to total wall touches.
Rotarod test:
   Mice were trained for 2 days before undergoing behavioral testing on the third day. On the first day, mice were trained four times on a rotarod with a fixed speed of 4 revolutions per minute (rpm) for 300 seconds each time. On the second day, mice were trained four times with an accelerating speed from 4 to 40 rpm. On the third day, behavioral testing was conducted with an accelerating speed from 4 to 40 rpm, with four trials in total. The time that the mouse stays on the bar before falling off is recorded as the duration of stay, and the average of the three longest durations of stay is used for analysis.
NMDA modeling:
   To investigate whether RGCs can regenerate in damaged retinas, a 200mM NMDA solution was prepared in PBS and then injected into the eyes of 4-8-week-old Ai9 mice or 5-6-week-old C57BL/6 mice (for VEP and black-and-white scene preference tests) through intravitreal injection. Two to three weeks after NMDA injection, GFAP-GFP-Cre and GFAP-CasRx-REST or GFAP-CasRx were co-delivered to the retina through subretinal injection. To assess the functional rescue of damaged retinas (VEP and light-dark box shuttle test), NMDA was injected into the eyes of 5-week-old to 12-month-old mice (C57BL/6) to induce retinal damage, and GFAP-mCherry (0.1 µl) mixed with PBS (0.9 µl) or GFAP-RZFD-V1 (0.9 µl)/GFAP-RZFD-V2 (0.9 µl)/GFAP-RZFD-V3 (0.9 µl) was injected subretinally two to three weeks later.
Subretinal AAV injection:
   High-titer (> 1×10¹³ vg/ml) AAV was injected into the eye using a Hamilton syringe (32G needle) under an Olympus microscope (Olympus, Tokyo, Japan). To determine reprogramming in the intact retina, a total of 1 µl of GFAP-GFP-Cre (0.1 µl) + PBS (0.9 µl), or GFAP-GFP-Cre (0.1 µl) and GFAP-RZFD-V1 (0.9 µl)/GFAP-RZFD-V2 (0.9 µl)/GFAP-RZFD-V3 (0.9 µl) was injected subretinally in Ai9 and C57BL/6 mice aged 4 weeks to 12 months.
Retina dissection and slicing:
   After 1-3 months of AAV injection, the eyes, optic nerves, and brain tissues were collected and fixed in 4% paraformaldehyde (PFA) for 2 hours (eyes and optic nerves) or 24 hours (brain). Subsequently, they were dehydrated in a 30% sucrose solution for 2 hours (eyes) or 24 hours (brain). The optic nerves were directly washed with PBS and mounted for observation. The eyes and brain were then embedded in OCT and sliced into sections with a thickness of 30 µm.
Immunofluorescence staining of the retina:
   The primary antibodies used for immunofluorescence staining were rabbit anti-RBPMS (1:500, 15187-1-AP, Proteintech), mouse anti-Brn3a (1:100, MAB1585, Millipore), rabbit anti-Sox9 (1:500, AB5535, Millipore), rabbit anti-Prox1 (1:500, AB5475, Millipore), and rabbit anti-Pax6 (1:500, 901301, Biolegend). The secondary antibodies were Cy^{™}5 AffiniPure Donkey anti-Mouse IgG (H + L) (1:500, 715-175-150, Jackson ImmunoResearch) and Cy^{™}5 AffiniPure Donkey anti-Rabbit IgG (H + L) (1:500, 711-175-152, Jackson ImmunoResearch). The primary antibodies were incubated overnight at 4°C and then washed three times with PBS for 10 minutes each time. The secondary antibodies were incubated for 2-3 hours at room temperature and then washed three times with PBS for 10 minutes each time. Finally, the sections were mounted with anti-fade mounting medium (Life technology) and imaged using an Olympus FV3000 microscope.
Statistical Analysis:
   Error bars were set by s.e.m., and statistical significance (p < 0.05) was calculated using unpaired two-tailed t-tests or one-way ANOVA. All experiments were conducted randomly, and the sample size was not pre-determined using statistical methods. It was assumed that the data distribution was normal, but formal tests were not performed. Data collection and analysis were not conducted under blinded experimental conditions.

### Example 1. Construction of tools targeting RE1

In order to realize the regulation of RE1, we drew upon the binding characteristics of REST and RE1, excavated the endogenous zinc finger protein (ZF) of the REST as a RE1 binding domain to regulate RE1, which is called RZFD (REST Zinc Finger Domain) (SEQ ID NO: 1). Through structure prediction and protein modeling, we found that there were eight zinc finger protein domains from 159 to 412 of the human REST protein (Fig. 1A). In order to further regulate the expression of nerve-related proteins controlled by RE1, we added a gene expression regulator VP64 (SEQ ID NO: 7) or P65-HSF1 (SEQ ID NO: 11) in the C-terminus of RZFD, which named RZFD- VP64 and RZFD-P65-HSF (Fig. 1A) respectively. VP64 and P65-HSF can recruit transcription factors and histone acetylation proteins to the vicinity of RE1, regulate the chromosome structure near RE1, and regulate the gene expression. In order to further optimize the RZFD system, we optimized the codon of RZFD (SEQ ID NO: 15) to increase the expression of RZFD in mammalian cells. At the same time, we added bpNLS sequences at both ends of RZFD to enhance the efficiency of entering into the nuclear. This version was named RZFDmax (Figure 1A). In non-neurons (such as: glial cells), the REST complex binds to RE1, and under the action of histone deacetylase and methylase in the complex, the chromatin near RE1 changes into a dense state, and the expression of neurons-associated gene was turned off (Figure 1B). We expressed RZFD, RZFD-VP64 or RZFD-P65-HSF1 in non-neuronal cells such as glial cells. RZFD, RZFD-VP64 or RZFD-P65-HSF1 could bind to RE1 competitively and prevents the REST silencing complex binding to RE1 (Fig. 1C, D and E). The mechanism of RZFD is competitive binding with RE1. The RZFD-VP64 and RZFD-P65-HSF1 can not only bind RE1 competitively, but also change the chromosomal state nearby RE1, and promote the expression of nerve-related genes which are regulated by RE1.

### Example 2. Construction of gene expression inhibition system

The REST protein could regulate the expression of REST targeting genes because the N-terminal (SEQ ID NO: 16) and C-terminal (SEQ ID NO: 18) of the REST protein have the function of recruiting various epigenetic regulatory elements. To investigate the ability of N-terminal and C-terminal to repress the expression of targeting genes, we constructed several different repressing systems and tested the repression efficiency of Ptbp1 in 293T cells (Fig. 1F-K). In order to compare the inhibition efficiency with Krab (SEQ ID NO: 28), which is a known gene expression inhibition domain, we also constructed dCas9-Krab (SEQ ID NO: 23) and dCas9-3 × Krab (SEQ ID NO: 24). Through the guidance of sgRNA after dCas9-Krab entering into the cell, dCas9-Krab binds to the regulatory region of endogenous gene expression to achieve the expression inhibition of the target genes. In order to study the difference of inhibition efficiencies of a single N-terminal, multiple N-terminals in series and both including N-terminal and C-terminal, we constructed N-dCas9 (SEQ ID NO: 25), 3 × N-dCas9 (SEQ ID NO: 26) and 3 × N-dCas9-3xC (SEQ ID NO: 27). These systems inhibit the expression of Ptbp 1 under the guidance of sgRNA targeting Ptbp1 (Figure 1G-K). In order to test the inhibition efficiency of various systems, we transfected them into 293T cells respectively, sorted the cells by flow cytometry, and tested the inhibition efficiency through RT-qPCR. The inhibition efficiencies of N-dCas9 and dCas9-Krab are quite close but relatively low. 3 × N-dCas9 is also not very efficient. Both dCas9-3 × Krab and 3 × N-dCas9-3xC have high efficiency especially 3 × N-dCas9-3xC (Fig. 1L). These results indicate that constructing repression system in the N-terminal and C-terminal of REST can effectively inhibit the expression of endogenous genes, and can be used as a tool for epigenetic regulation or gene expression inhibition.

### Example 3. Use of RZFD and RZFDmax to trans-differentiate astrocytes into dopamine neurons

In order to trans-differentiate the glial cells into neuronal cells in mice, we constructed an AAV expression vector. We used the astrocyte-specific promoter GFAP to drive the expression of the fluorescent protein mCherry (GFAP-mCherry). The human RZFD was also driven by the GFAP promoter (GFAP -RZFD, wherein the amino acid sequence of RZFD is SEQ ID NO: 1, and the nucleic acid sequence is SEQ ID NO: 2) which is specifically expressed in astrocytes (FIG. 2A). To investigate whether glial cells can be induced to transdifferentiate into neurons or dopaminergic neurons at 2 weeks or longer after injection, we collected samples at 2 weeks or in 1.5 months after the AAV injection respectively, and performed staining for the neuron-specific marker NeuN and the dopaminergic neuron-specific marker Tyrosine hydroxylase (TH) (Figure 2B). The 8-week-old C57 mice were injected with the AAV expressing GFAP-mCherry + GFAP-RZFD into the striatum or substantia nigra, which is named as the test group. The 8-week-old C57 mice were injected with the AAV expressing GFAP-mCherry alone which is named as the control group (Fig. 2C). Our previous studies showed that no neurons were generated in the control group from 1 week to 3 months after the injection. Consistent with previous findings, we did not observe the generation of neurons two weeks after injection in the control group (Fig. 2D). In the test group, some cells began to change shape and a small number of glial cells began to express the neuron-specific marker NeuN 2 weeks after injection (Fig. 2E). This indicated that expressing RZFD in astrocytes has the potential to transdifferentiate astrocytes into neurons. Analyzed the test group at 1.5 months following the injection, we found a large number of mCherry⁺NeuN⁺ double-positive cells, while no mCherry⁺NeuN⁺ double-positive cells appeared in the control group. This indicated that RZFD can efficiently trans-differentiate the astrocytes into neurons. At the same time, we detected the expression of TH which is a specific marker for detecting dopamine neurons, and found that the expression of TH was enhanced and neurons were formed in some mCherry-positive cells. These indicated that RZFD could transdifferentiate astrocytes into dopamine neurons (Fig. 2F). In order to investigate whether RZFDmax can further enhance the efficiency of trans-differentiation of astrocytes into neurons and dopamine neurons, the mixed AAV virus of GFAP-mCherry + GFAP-RZFDmax was injected in the striatum of mice, and the differentiation efficiency was analyzed. A number of TH-positive neurons was found in the striatum of mice after injected with GFAP-mCherry + GFAP-RZFDmax (Fig. 2G). Through statistical analysis, the number of TH positive neurons generated in the GFAP-RZFDmax group was significantly more than that in the GFAP-RZFD group (Fig. 2H). These results indicated that both GFAP-RZFD and GFAP-RZFDmax could induce the trans-differentiation of astrocytes into neurons and dopamine neurons. GFAP-RZFDmax induced the trans-differentiation of glial cells into dopamine neurons more efficiently.

### Example 4. RZFD alleviates the symptoms in a Parkinson's mouse model for trans-differentiating astrocytes into dopamine neurons

In order to further investigate whether GFAP-RZFD can alleviate the symptoms of Parkinson's disease in mouse model, we first injected 6-OHDA into the MFB of Dat-Cre:Ai9 mice to establish the model. Then, the AAV of the control group and the test group were injected into the striatum (ST) or substantia nigra (Nigra) of the mice model, and analyzed 1.5-3 months after injection (Fig. 3A). Dat-Cre: Ai9 can specifically label dopamine neurons, and only dopamine neurons will be labeled as tdTomato positive. In general, there are no positive cells in the mouse striatum, and if there are glial cells transdifferentiated into dopamine neurons, they will be marked in red. In the control group, we injected AAV expressing GFAP-EGFP, the EGFP green fluorescence signal was specifically expressed in astrocytes, these cells maintained the typical astrocyte morphology, no red signal was found under red fluorescence signal, no TH-positive cells were found. These results indicated that the GFAP-EGFP control group did not transdifferentiate to produce dopamine neurons (Fig. 3B). In the test group injected with GFAP-EGFP + GFAP-RZFD, some EGFP-positive cells were observed to transdifferentiate into neurons, and a number of tdTomato-positive cells appeared. TH staining showed that these tdTomato-positive cells colocalized with TH (Figure 3C). This suggests that overexpression of RZFD in Dat-Cre: Ai9 mice can transdifferentiate glial cells into dopamine neurons. To further test whether these transdifferentiated neurons can alleviate the symptoms in the Parkinson's mouse model, we performed the rotarod test, the cylinder test and the drug-induced rotational behavior test on the mice (Fig. 3D-G). In the rotarod test, the mice in the control group could maintain about 120s, while the treated mice could reach about 180s (Figure 3D). In the cylinder test, the balance of the front paws of the mice in the test group was significantly better than that in the control group (Fig. 3E). In the drug-induced rotational behavior test, the test group showed a significant reduction in circling behavior relative to the control group (Fig. 3F and 3G). These results indicate that transdifferentiated dopamine neurons can significantly alleviate the disease symptoms in the Parkinson's mouse model.

### Example 5. RZFD-VP64 and RZFD-P65-HSF1 transdifferentiate astrocytes into dopamine neurons

In order to further study the regulation of RE1 neuron-related gene expression, we constructed AAV expression vector of RZFD-VP64 (annotated as GFAP-RZFD-V2, amino acid sequence SEQ ID NO: 5, nucleic acid sequence SEQ ID NO: 6) and RZFD-P65-HSF1(annotated as GFAP-RZFD-V3, amino acid sequence SEQ ID NO: 9, nucleic acid sequence SEQ ID NO: 10) (Fig. 4A). Driven by the glial cell-specific promoter GFAP, RZFD-VP64 and RZFD-P65-HSF1 were expressed. C57 mice were injected with the mixed AAV of GFAP-mCherry + GFAP-RZFD-VP64 into the striatum or substantia nigra, and the samples were got and analyzed after 1.5 months (Fig. 4B). We found that most of the red fluorescently labeled cells in the group GFAP-RZFD-VP64 expressed the neuron-specific marker NeuN, and some cells expressed the dopamine neuron-specific marker TH (Fig. 4C). We performed similar AAV injections in the striatum or substantia nigra of DAT-Cre: Ai9 mice. The mice were injected with GFAP-RZFD-P65-HSF1 into the striatum or substantia nigra, and harvested 1.5 months later analysis (Fig. 4D). In DAT-Cre: Ai9 mice, only mature dopamine neurons could be labeled. Our study found no red fluorescent labeled cells in the striatum of the mice in control group. However, DAT-Cre: Ai9 mice injected with GFAP-RZFD-P65-HSF1 produced red cells in the striatum. After NeuN and TH staining, we found that these cells with red fluorescent signals not only expressed neuron-specific markers NeuN, but also expressed the dopamine neuron-specific marker TH (Fig. 4E). This suggests that expression of RZFD-P65-HSF1 in glial cells can transdifferentiate astrocytes into dopamine neurons.

### Example 6. RZFD transdifferentiates Müller glial cells into photoreceptor cells and retinal ganglion cells

In order to study the role of RZFD in the mouse retina, we injected AAV expressing NLS-RZFD-V1 (SEQ ID NO: 3) into the mouse retina to explore whether RZFD can transdifferentiate Müller glia cells in the retina into other types of cells. In Ai9 mice, AAV expressing GFAP-EGFP-2A-Cre was delivered into the eyes of mice by subretinal injection. GFAP-EGFP-2A-Cre is used to label Müller glial cells. Under the action of GFAP promoter, Cre is specifically expressed in Müller glial cells, which unwrap the LSL sequence in Ai9 mice, thereby achieving the purpose of labeling Müller glial cells in Ai9 mice. (Figure 5A). First, we conducted NMDA modeling in Ai9 mice, AAV was injected subsequently. In the control group, there was no obvious deformation of the labeled Müller glia after injection of GFAP-EGFP-2A-Cre, and there were no cells other than Müller glia to be labeled (Fig. 5B). In contrast, in the group injected with AAV expressing NLS-RZFD-V1(i.e.: GFAP-RZFD), tdTomato-positive photoreceptor cells were observed in the outer granule cell layer, and many Müller glia cells were deformed (Fig. 5C). Staining with photoreceptor cell-specific protein markers Rhodopsin and Cone arrestin revealed that tdTomato-positive cells located in the outer granular layer were co-labeled with Rhodopsin or Cone arrestin (i.e., MCAR) (Figure 5H and 5I). This suggests that overexpression of RZFD in Müller glial cells can transdifferentiate them into photoreceptor cells. The number of photoreceptor cells was counted, and it was found that the average number of photoreceptor cells in each visual field was about 8, while there were almost no tdTomato-positive photoreceptor cells to be observed in the control group (Fig. 5J). In order to test whether the overexpression of RZFD can transdifferentiate Müller glial cells into retinal ganglion cells, we took the mouse optic nerve after injecting and scanned the whole tissue. there were almost no tdTomato-positive axons in the control group, while there were a large number of tdTomato-positive axons in the test group (Fig. 5D and 5E). Further Rbpms staining was performed on the retina, all tdTomato-positive cells were located in the inner granule cell layer in the control group, there were no tdTomato-positive cells in the retinal ganglion cell layer. In the RZFD overexpression group, some tdTomato-positive cells migrated to the retinal ganglion cells layer, and was co-labeled with a retinal ganglion cell-specific protein marker Rbpms (Fig. 5F and 5G). Statistical analysis of optic nerves revealed that there were more than 50 axons to be labeled with tdTomato in each optic nerve (Fig. 5K). This suggests that RZFD overexpression in Müller glia can also transdifferentiate Müller glia into retinal ganglion cells. In summary, overexpression of RZFD can not only transdifferentiate Müller glial cells into retinal ganglion cells, but also transdifferentiate into photoreceptor cells.

### Example 7. Overexpression of miR-9 or miR-124 cannot transdifferentiate glial cells into neurons

Previous studies have shown that knocking down Ptbp1 can transdifferentiate glial cells into dopamine neurons. Knocking down Ptbp1 can upregulate the expression of miR-124, which has been reported to downregulate the expression of REST. In order to study whether miR-124 can effectively reduce the expression of REST, we overexpressed miR-124 (nucleic acid coding sequence SEQ ID NO: 31) in 293T and N2A cells to verify whether miR-124 overexpression can effectively reduce the expression of REST protein.

We used an AAV vector and used the tissue-specific promoter GFAP to promote the expression of miR-124 pri-miRNA (as shown in SEQ ID NO: 34), which will be processed into pre-miRNA ( shown in SEQ ID NO: 33), and finally processed into mature miRNA (SEQ ID NO: 31 and SEQ ID NO: 32), so as to achieve tissue-specific expression of miR-124. The experimental results found that overexpression of miR-124 could not effectively reduce the expression of REST.

In order to further study, we used the same method in glial cells, using a similar AAV vector to promote the overexpression of miR-124 with the tissue-specific promoter GFAP, to verify whether it can transdifferentiate glial cells into Neurons. Analysis the brain sections from the injected mouse, it was found that overexpression of miR-124 did not transdifferentiate glial cells into neurons (Fig. 6A-D). In the control group, the red fluorescent signal mCherry was expressed in glial cells, and it was not co-labeled with the neuron-specific marker NeuN, and there were also no TH-positive neurons in the striatum (Fig. 6C). Therefore, upregulation of miR-124 alone is not sufficient to achieve trans-differentiation of glial cells into dopamine neurons.

Previous studies have also shown that Ptbp2 can promote the trans-differentiation of fibroblasts into neurons in vitro by regulating the expression of miR-9. Overexpression of miR-9 can promote the maturation of neurons differentiated in vitro. In order to study whether the overexpression of miR-9 can effectively promote the trans-differentiation of glial cells into dopamine neurons. We constructed an AAV vector for the overexpression of GFAP-miR-9, and delivered it to the striatum of mice by AAV Under the action of the GFAP promoter, miR-9 pri-miRNA (as shown in SEQ ID NO: 38) expressed in glial cells specifically, miR-9 pri-miRNA will be processed into pre-miRNA (as shown in SEQ ID NO: 37) in the cell after expression, and finally processed into mature miRNA (SEQ ID NO: 35 and SEQ ID NO: 36), thereby achieving tissue-specific expression of miR-9. At the same time, GFAP-mCherry was used to label astrocytes in the striatum.

Analysis through section staining has revealed that overexpression of miR-9 does not promote the trans-differentiation of glial cells into neurons. Similar to miR-124, overexpression of either miR-9 or miR-124 was unable to transdifferentiate astrocytes into neurons or dopamine neurons although the morphology of glial cells had been changed.

### Example 8. Co-overexpression of miR-9 and miR124 can transdifferentiate glial cells into neurons

Previous studies in vitro have shown that the co-overexpression of miR-124 and miR-9 can promote the differentiation of stem cells into neurons. We drove miR-9 and miR124 with a GFAP promoter, packaged them into AAV, and injected them into the striatum of mice to study whether the co-overexpression of miR-124 and miR-9 could promote the transition from glial cells to neurons. We surprisingly found that co-overexpression of miR-9 and miR124 could transdifferentiate glial cells into neurons (Fig. 7E). Further staining with the dopamine-specific protein marker TH revealed that co-overexpression of miR-9 and miR124 could transdifferentiate glial cells into neurons, but could not transdifferentiate glial cells into dopamine neurons (Fig. 7F).

### Example 9 Using CasRx to specifically knock down Ctdsp1 in vitro

Previous studies shown that knocking down the expression of Ctdsp1 (amino acid sequence shows as SEQ ID NO:29, nucleic acid sequence shows as SEQ ID NO:30) can promote the trans-differentiation of fibroblasts into neurons in vitro. The previous study used the shRNA to knock down. CasRx-mediated RNA editing technology is a new generation of RNA knockdown technology with higher efficiency and stronger specificity. In order to determine the efficiency of CasRx-mediated Ctdsp1 knockdown, a gRNA targeting Ctdsp1 was designed, and the gRNA targeting region was the 318-347 positions of the CDS sequence of the Ctdsp1 gene. Their inhibition efficiencies were compared in human 293T and mouse N2A cells. The plasmids transfected in the control group were CAG-CasRx-P2A-GFP and U6-nontarget-CMV-mCherry. The plasmids transfected in the test group were CAG-CasRx-P2A-GFP and U6-gRNA (Ctdsp1)-CMV-mCherry. Positive cells were sorted by flow cytometry after transfection and analyzed by QPCR. The results showed that co-transfection of gRNA targeting Ctdsp1 mRNA and CasRx could efficiently knock down the expression of Ctdsp1 in human 293T and mouse N2A cells (Figure 8).

### Example 10. Knockdown of Ctdsp1 in astrocytes transdifferentiates glial cells into neurons but fails to generate dopamine neurons

In order to further investigate whether knocking down Ctdsp1 in astrocytes can transdifferentiate glial cells into neurons in vivo, we constructed GFAP-CasRx-gRNA(Ctdsp1) and used GFAP-CasRx AAV vector without gRNA as a control, while labeling glial cells with GFAP-mCherry. (Fig. 9A). The sections were analyzed in 1-2 months after AAV virus injection. In the control group, GFAP-mCherry-labeled astrocytes still maintained the typical glial cell morphology, and did not co-labelled with the neuron-specific marker NeuN. marked (Fig. 9B). In the group of Ctdsp1 knockdown, we found that some cells expressing red fluorescent signal expressed NeuN, which indicated that knockdown of Ctdsp1 could transdifferentiate astrocytes into neurons (Fig. 9C). It was the first to demonstrate that Ctdsp1 could be a target for the trans-differentiation of astrocytes into neurons in vivo. Further staining with the dopamine-specific protein marker TH revealed that knockdown of Ctdsp1 did not transdifferentiate astrocytes into dopamine neurons in vivo.

### Example 11. Knockdown of Ctdsp1 or overexpression of miR-9 or miR-124 in the retina cannot transdifferentiate glial cells into photoreceptor cells or retinal ganglion cells

In order to study whether knocking down Ctdsp1 or overexpressing miR-9 or miR-124 can transdifferentiate Müller glial cells in the retina into retinal ganglion cells or photoreceptor cells, GFAP-gRNA (Ctdsp1) or miR-9 or miR-124 or miR-9 + miR-124 were injected into the retina of Ai9 mice respectively, GFAP-EGFP-2A-Cre was injected at the same time to label Müller glial cells. Analysis in 1-2 months after injection, it was showed that neither knockdown of Ctdsp1 nor overexpression of miR-9 or miR-124 or miR-9 + miR-124 could transdifferentiate Müller glia cells into retinal ganglion cells or photoreceptor cells (Fig. 10). These results suggest that overexpression of miR-9 or miR-124 alone cannot transdifferentiate glial cells into neurons, whereas knockdown of Ctdsp1 or overexpression of miR-9+miR-124 can transdifferentiate astrocytes into common neurons in the brain but cannot produce the specific types of neurons, such as dopamine neurons, photoreceptor cells and retinal ganglion cells.

### SEQUENCES INFORMATION

**Amino acid sequence of human RZFD (SEQ ID NO: 1)** 
KTKPFRCKPCQYEAESEEQFVHHIRVHSAKKFFVEESAEKQAKARESGSSTAEEGDFSKGP
IRCDRCGYNTNRYDHYTAHLKHHTRAGDNERVYKCIICTYTTVSEYHWRKHLRNHFPRK
VYTCGKCNYFSDRKNNYVQHVRTHTGERPYKCELCPYSSSQKTHLTRHMRTHSGEKPFK
CDQCSYVASNQHEVTRHARQVHNGPKPLNCPHCDYKTADRSNFKKHVELHVNPRQFNC
PVCDYAASKKCNLQYHFKSKHPTCPNKTM
**Coding sequence of human RZFD (SEQ ID NO: 2)** 
aagaccaaaccctttcgctgtaagccatgccaatatgaagcagaatctgaagaacagtttgtgcatcacatcagagttcacagtgctaagaaatt
ttttgtggaagagagtgcagagaagcaggcaaaagccagggaatctggctcttccactgcagaagagggagatttctccaagggccccattc
gctgtgaccgctgcggctacaatactaatcgatatgatcactatacagcacacctgaaacaccacaccagagctggggataatgagcgagtct
acaagtgtatcatttgcacatacacaacagtgagcgagtatcactggaggaaacatttaagaaaccattttccaaggaaagtatacacatgtgg
aaaatgcaactatttttcagacagaaaaaacaattatgttcagcatgttagaactcatacaggagaacgcccatataaatgtgaactttgtccttac
tcaagttctcagaagactcatctaactagacatatgcgtactcattcaggtgagaagccatttaaatgtgatcagtgcagttatgtggcctctaatc
aacatgaagtaacccgccatgcaagacaggttcacaatgggcctaaacctcttaattgcccacactgtgattacaaaacagcagatagaagca
acttcaaaaaacatgtagagctacatgtgaacccacggcagttcaattgccctgtatgtgactatgcagcttccaagaagtgtaatctacagtatc
acttcaaatctaagcatcctacttgtcctaataaaacaatg
**Amino acid sequence of NLS-RZFD-V1 (SEQ ID NO: 3)** 
MPKKKRKVGSGKTKPFRCKPCQYEAESEEQFVHHIRVHSAKKFFVEESAEKQAKARESGS
STAEEGDFSKGPIRCDRCGYNTNRYDHYTAHLKHHTRAGDNERVYKCIICTYTTVSEYHW
RKHLRNHFPRKVYTCGKCNYFSDRKNNYVQHVRTHTGERPYKCELCPYSSSQKTHLTRH
MRTHSGEKPFKCDQCSYVASNQHEVTRHARQVHNGPKPLNCPHCDYKTADRSNFKKHV
ELHVNPRQFNCPVCDYAASKKCNLQYHFKSKHPTCPNKTM
**Nucleotide sequence of NLS-RZFD-V1 (SEQ ID NO: 4)** 
atgCCTAAAAAGAAAAGAAAGGTGGGTTCTGGTaagaccaaaccctttcgctgtaagccatgccaatatgaagc
agaatctgaagaacagtttgtgcatcacatcagagttcacagtgctaagaaattttttgtggaagagagtgcagagaagcaggcaaaagccag
ggaatctggctcttccactgcagaagagggagatttctccaagggccccattcgctgtgaccgctgcggctacaatactaatcgatatgatcac
tatacagcacacctgaaacaccacaccagagctggggataatgagcgagtctacaagtgtatcatttgcacatacacaacagtgagcgagtat
cactggaggaaacatttaagaaaccattttccaaggaaagtatacacatgtggaaaatgcaactatttttcagacagaaaaaacaattatgttcag
catgttagaactcatacaggagaacgcccatataaatgtgaactttgtccttactcaagttctcagaagactcatctaactagacatatgcgtactc
 
attcaggtgagaagccatttaaatgtgatcagtgcagttatgtggcctctaatcaacatgaagtaacccgccatgcaagacaggttcacaatgg
gcctaaacctcttaattgcccacactgtgattacaaaacagcagatagaagcaacttcaaaaaacatgtagagctacatgtgaacccacggca
gttcaattgccctgtatgtgactatgcagcttccaagaagtgtaatctacagtatcacttcaaatctaagcatcctacttgtcctaataaaacaatg
**Amino acid sequence of NLS-RZFD-V2 (SEQ ID NO: 5)** 
MPKKKRKVGSGKTKPFRCKPCQYEAESEEQFVHHIRVHSAKKFFVEESAEKQAKARESGS
STAEEGDFSKGPIRCDRCGYNTNRYDHYTAHLKHHTRAGDNERVYKCIICTYTTVSEYHW
RKHLRNHFPRKVYTCGKCNYFSDRKNNYVQHVRTHTGERPYKCELCPYSSSQKTHLTRH
MRTHSGEKPFKCDQCSYVASNQHEVTRHARQVHNGPKPLNCPHCDYKTADRSNFKKHV
ELHVNPRQFNCPVCDYAASKKCNLQYHFKSKHPTCPNKTMGSGGGGSGGGGSRADALD
DFDLDMLGSDALDDFDLDMLGSDALDDFDLDMLGSDALDDFDLDMLYID
**Nucleotide sequence of NLS-RZFD-V2 (SEQ ID NO: 6)** 
atgCCTAAAAAGAAAAGAAAGGTGGGTTCTGGTaagaccaaaccctttcgctgtaagccatgccaatatgaagc
agaatctgaagaacagtttgtgcatcacatcagagttcacagtgctaagaaattttttgtggaagagagtgcagagaagcaggcaaaagccag
ggaatctggctcttccactgcagaagagggagatttctccaagggccccattcgctgtgaccgctgcggctacaatactaatcgatatgatcac
tatacagcacacctgaaacaccacaccagagctggggataatgagcgagtctacaagtgtatcatttgcacatacacaacagtgagcgagtat
cactggaggaaacatttaagaaaccattttccaaggaaagtatacacatgtggaaaatgcaactatttttcagacagaaaaaacaattatgttcag
catgttagaactcatacaggagaacgcccatataaatgtgaactttgtccttactcaagttctcagaagactcatctaactagacatatgcgtactc
attcaggtgagaagccatttaaatgtgatcagtgcagttatgtggcctctaatcaacatgaagtaacccgccatgcaagacaggttcacaatgg
gcctaaacctcttaattgcccacactgtgattacaaaacagcagatagaagcaacttcaaaaaacatgtagagctacatgtgaacccacggca
gttcaattgccctgtatgtgactatgcagcttccaagaagtgtaatctacagtatcacttcaaatctaagcatcctacttgtcctaataaaacaatg
GGTAGTGGCGGTGGTGGTTCAGGAGGCGGCGGAAGCCGCGCCGACGCGCTGGACGAT
TTCGATCTCGACATGCTGGGTTCTGATGCCCTCGATGACTTTGACCTGGATATGTTGGG
AAGCGACGCATTGGATGACTTTGATCTGGACATGCTCGGCTCCGATGCTCTGGACGATT
TCGATCTCGATATGTTATATATCGAT
**Amino acid sequence of VP64 (SEQ ID NO: 7)**
   RADALDDFDLDMLGSDALDDFDLDMLGSDALDDFDLDMLGSDALDDFDLDMLYID
**Nucleotide sequence of VP64 (SEQ ID NO: 8)** 
CGCGCCGACGCGCTGGACGATTTCGATCTCGACATGCTGGGTTCTGATGCCCTCGATGA
CTTTGACCTGGATATGTTGGGAAGCGACGCATTGGATGACTTTGATCTGGACATGCTCG
GCTCCGATGCTCTGGACGATTTCGATCTCGATATGTTATATATCGAT
**Amino acid sequence of NLS-RZFD-V3 (SEQ ID NO: 9)** 
MPKKKRKVGSGKTKPFRCKPCQYEAESEEQFVHHIRVHSAKKFFVEESAEKQAKARESGS
STAEEGDFSKGPIRCDRCGYNTNRYDHYTAHLKHHTRAGDNERVYKCIICTYTTVSEYHW
RKHLRNHFPRKVYTCGKCNYFSDRKNNYVQHVRTHTGERPYKCELCPYSSSQKTHLTRH
MRTHSGEKPFKCDQCSYVASNQHEVTRHARQVHNGPKPLNCPHCDYKTADRSNFKKHV
ELHVNPRQFNCPVCDYAASKKCNLQYHFKSKHPTCPNKTMGSGGGGSGGGGSRADALD
DFDLDMLGSDALDDFDLDMLGSDALDDFDLDMLGSDALDDFDLDMLYIDDYKLGSGGG
GSGGGGSPSGQISNQALALAPSSAPVLAQTMVPSSAMVPLAQPPAPAPVLTPGPPQSLSAP
VPKSTQAGEGTLSEALLHLQFDADEDLGALLGNSTDPGVFTDLASVDNSEFQQLLNQGVS
 
MSHSTAEPMLMEYPEAITRLVTGSQRPPDPAPTPLGTSGLPNGLSGDEDFSSIADMDFSALL
SQISSSGQGGGGSGFSVDTSALLDLFSPSVTVPDMSLPDLDSSLASIQELLSPQEPPRPPEAE
NSSPDSGKQLVHYTAQPLFLLDPGSVDTGSNDLPVLFELGEGSYFSEGDGFAEDPTISLLTG
SEPPKAKDPTVSPKKKRKV
**Nucleotide sequence of NLS-RZFD-V3 (SEQ ID NO: 10)** 
atgCCTAAAAAGAAAAGAAAGGTGGGTTCTGGTaagaccaaaccctttcgctgtaagccatgccaatatgaagc
agaatctgaagaacagtttgtgcatcacatcagagttcacagtgctaagaaattttttgtggaagagagtgcagagaagcaggcaaaagccag
ggaatctggctcttccactgcagaagagggagatttctccaagggccccattcgctgtgaccgctgcggctacaatactaatcgatatgatcac
tatacagcacacctgaaacaccacaccagagctggggataatgagcgagtctacaagtgtatcatttgcacatacacaacagtgagcgagtat
cactggaggaaacatttaagaaaccattttccaaggaaagtatacacatgtggaaaatgcaactatttttcagacagaaaaaacaattatgttcag
catgttagaactcatacaggagaacgcccatataaatgtgaactttgtccttactcaagttctcagaagactcatctaactagacatatgcgtactc
attcaggtgagaagccatttaaatgtgatcagtgcagttatgtggcctctaatcaacatgaagtaacccgccatgcaagacaggttcacaatgg
gcctaaacctcttaattgcccacactgtgattacaaaacagcagatagaagcaacttcaaaaaacatgtagagctacatgtgaacccacggca
gttcaattgccctgtatgtgactatgcagcttccaagaagtgtaatctacagtatcacttcaaatctaagcatcctacttgtcctaataaaacaatg
GGTAGTGGCGGTGGTGGTTCAGGAGGCGGCGGAAGCCGCGCCGACGCGCTGGACGAT
TTCGATCTCGACATGCTGGGTTCTGATGCCCTCGATGACTTTGACCTGGATATGTTGGG
AAGCGACGCATTGGATGACTTTGATCTGGACATGCTCGGCTCCGATGCTCTGGACGATT
TCGATCTCGATATGTTATATATCGATGATTACAAGCTTGGTAGTGGCGGTGGTGGTTCAG
GAGGCGGCGGAAGCCCTTCAGGGCAGATCAGCAACCAGGCCCTGGCTCTGGCCCCTA
GCTCCGCTCCAGTGCTGGCCCAGACTATGGTGCCCTCTAGTGCTATGGTGCCTCTGGCC
CAGCCACCTGCTCCAGCCCCTGTGCTGACCCCAGGACCACCCCAGTCACTGAGCGCTC
CAGTGCCCAAGTCTACACAGGCCGGCGAGGGGACTCTGAGTGAAGCTCTGCTGCACC
TGCAGTTCGACGCTGATGAGGACCTGGGAGCTCTGCTGGGGAACAGCACCGATCCCG
GAGTGTTCACAGATCTGGCCTCCGTGGACAACTCTGAGTTTCAGCAGCTGCTGAATCA
GGGCGTGTCCATGTCTCATAGTACAGCCGAACCAATGCTGATGGAGTACCCCGAAGCC
ATTACCCGGCTGGTGACCGGCAGCCAGCGGCCCCCCGACCCCGCTCCAACTCCCCTGG
GAACCAGCGGCCTGCCTAATGGGCTGTCCGGAGATGAAGACTTCTCAAGCATCGCTGA
TATGGACTTTAGTGCCCTGCTGTCACAGATTTCCTCTAGTGGGCAGGGAGGAGGTGGA
AGCGGCTTCAGCGTGGACACCAGTGCCCTGCTGGACCTGTTCAGCCCCTCGGTGACCG
TGCCCGACATGAGCCTGCCTGACCTTGACAGCAGCCTGGCCAGTATCCAAGAGCTCCT
GTCTCCCCAGGAGCCCCCCAGGCCTCCCGAGGCAGAGAACAGCAGCCCGGATTCAGG
GAAGCAGCTGGTGCACTACACAGCGCAGCCGCTGTTCCTGCTGGACCCCGGCTCCGTG
GACACCGGGAGCAACGACCTGCCGGTGCTGTTTGAGCTGGGAGAGGGCTCCTACTTC
TCCGAAGGGGACGGCTTCGCCGAGGACCCCACCATCTCCCTGCTGACAGGCTCGGAG
CCTCCCAAAGCCAAGGACCCCACTGTCTCCCCCAAGAAGAAGCGCAAGGTG
**Amino acid sequence of P65-HSF1 (SEQ ID NO: 11)** 
PSGQISNQALALAPSSAPVLAQTMVPSSAMVPLAQPPAPAPVLTPGPPQSLSAPVPKSTQA
GEGTLSEALLHLQFDADEDLGALLGNSTDPGVFTDLASVDNSEFQQLLNQGVSMSHSTAE
PMLMEYPEAITRLVTGSQRPPDPAPTPLGTSGLPNGLSGDEDFSSIADMDFSALLSQISSSG
QGGGGSGFSVDTSALLDLFSPSVTVPDMSLPDLDSSLASIQELLSPQEPPRPPEAENSSPDS
GKQLVHYTAQPLFLLDPGSVDTGSNDLPVLFELGEGSYFSEGDGFAEDPTISLLTGSEPPKA
KDPTVS
**Nucleotide sequence of P65-HSF1 (SEQ ID NO: 12)** 
CCTTCAGGGCAGATCAGCAACCAGGCCCTGGCTCTGGCCCCTAGCTCCGCTCCAGTGC
TGGCCCAGACTATGGTGCCCTCTAGTGCTATGGTGCCTCTGGCCCAGCCACCTGCTCCA
GCCCCTGTGCTGACCCCAGGACCACCCCAGTCACTGAGCGCTCCAGTGCCCAAGTCTA
CACAGGCCGGCGAGGGGACTCTGAGTGAAGCTCTGCTGCACCTGCAGTTCGACGCTG
ATGAGGACCTGGGAGCTCTGCTGGGGAACAGCACCGATCCCGGAGTGTTCACAGATCT
GGCCTCCGTGGACAACTCTGAGTTTCAGCAGCTGCTGAATCAGGGCGTGTCCATGTCT
CATAGTACAGCCGAACCAATGCTGATGGAGTACCCCGAAGCCATTACCCGGCTGGTGA
CCGGCAGCCAGCGGCCCCCCGACCCCGCTCCAACTCCCCTGGGAACCAGCGGCCTGC
CTAATGGGCTGTCCGGAGATGAAGACTTCTCAAGCATCGCTGATATGGACTTTAGTGCC
CTGCTGTCACAGATTTCCTCTAGTGGGCAGGGAGGAGGTGGAAGCGGCTTCAGCGTG
GACACCAGTGCCCTGCTGGACCTGTTCAGCCCCTCGGTGACCGTGCCCGACATGAGCC
TGCCTGACCTTGACAGCAGCCTGGCCAGTATCCAAGAGCTCCTGTCTCCCCAGGAGCC
CCCCAGGCCTCCCGAGGCAGAGAACAGCAGCCCGGATTCAGGGAAGCAGCTGGTGCA
CTACACAGCGCAGCCGCTGTTCCTGCTGGACCCCGGCTCCGTGGACACCGGGAGCAA
CGACCTGCCGGTGCTGTTTGAGCTGGGAGAGGGCTCCTACTTCTCCGAAGGGGACGG
CTTCGCCGAGGACCCCACCATCTCCCTGCTGACAGGCTCGGAGCCTCCCAAAGCCAAG
GACCCCACTGTCTCC
**Amino acid sequence of BPNLS (SEQ ID NO: 13)**
   KRTADGSEFESPKKKRKV
**Nucleotide sequence of BPNLS (SEQ ID NO: 14)**
   AAACGGACAGCCGATGGCAGCGAGTTCGAGAGCCCCAAGAAGAAGAGAAAGGTG
**Codon optimized RZFD (SEQ ID NO: 15)** 
AAGACCAAGCCCTTCAGGTGTAAACCCTGCCAGTACGAGGCCGAGTCTGAGGAACAG
TTTGTCCACCACATCAGAGTGCACAGCGCTAAGAAATTCTTCGTTGAGGAATCTGCCG
AGAAGCAGGCCAAGGCCCGGGAAAGCGGCTCCAGTACAGCCGAAGAGGGCGACTTC
AGCAAGGGCCCTATCCGGTGCGACAGATGTGGCTATAATACCAACAGATACGACCACT
ACACAGCGCACCTGAAGCACCACACCCGGGCCGGCGACAACGAGAGAGTGTACAAG
TGCATCATCTGCACCTACACCACAGTGTCCGAGTACCACTGGCGGAAACATCTCAGAA
ACCACTTTCCAAGAAAGGTGTACACCTGCGGCAAGTGCAACTACTTCAGCGACCGGA
AGAACAACTACGTGCAGCACGTGCGGACACACACCGGCGAGAGACCTTATAAGTGTG
AACTGTGCCCTTACAGCAGCAGCCAGAAAACTCACCTGACCCGCCACATGAGAACCC
ATAGCGGAGAAAAGCCATTTAAGTGCGACCAGTGCAGCTACGTGGCCAGCAATCAACA
CGAGGTGACCAGACACGCCAGACAGGTGCACAACGGCCCTAAGCCTCTGAACTGTCC
CCACTGCGACTACAAAACCGCCGATCGGAGCAACTTCAAGAAACACGTGGAACTGCA
CGTGAATCCTAGACAGTTCAACTGCCCCGTGTGCGATTACGCCGCCTCCAAGAAGTGT
AACCTGCAATACCACTTCAAGAGCAAGCATCCTACATGCCCTAACAAGACCATG
**Amino acid sequence of the N-terminal of human REST (SEQ ID NO: 16)** MATQVMGQSSGGGGLFTSSGNIGMALPNDMYDLHDLSKAELAAPQLIMLANVALTGEV NGSCCDYLVGEERQMAELMPVGDNNFSDSEEGEGLEESADIKGEPHGLENMELR
**Nucleotide sequence of the N-terminal of human REST (SEQ ID NO: 17)** 
atggccacccaggtaatggggcagtcttctggaggaggagggctgtttaccagcagtggcaacattggaatggccctgcctaacgacatgta
tgacttgcatgacctttccaaagctgaactggccgcacctcagcttattatgctggcaaatgtggccttaactggggaagtaaatggcagctgct
gtgattacctggtcggtgaagaaagacagatggcagaactgatgccggttggggataacaacttttcagatagtgaagaaggagaaggactt
gaagagtctgctgatataaaaggtgaacctcatggactggaaaacatggaactgaga
**Amino acid sequence of the C-terminal of human REST (SEQ ID NO: 18)** 
MDEGIHSHEGSDLSDNMSEGSDDSGLHGARPVPQESSRKNAKEALAVKAAKGDFVCIFC
DRSFRKGKDYSKHLNRHLVNVYYLEEAAQGQE
**Nucleotide sequence of the C-terminal of human REST (SEQ ID NO: 19)** 
ATGgatgaaggcatccacagccatgaaggaagtgacctaagtgacaacatgtcagagggtagtgatgattctggattgcatggggctcgg
ccagttccacaagaatctagcagaaaaaatgcaaaggaagccttggcagtcaaagcggctaagggagattttgtttgtatcttctgtgatcgttc
tttcagaaagggaaaagattacagcaaacacctcaatcgccatttggttaatgtgtactatcttgaagaagcagctcaagggcaggag
**Amino acid sequence of the full human REST (SEQ ID NO: 20)** 
MATQVMGQSSGGGGLFTSSGNIGMALPNDMYDLHDLSKAELAAPQLIMLANVALTGEV
NGSCCDYLVGEERQMAELMPVGDNNFSDSEEGEGLEESADIKGEPHGLENMELRSLELSV
VEPQPVFEASGAPDIYSSNKDLPPETPGAEDKGKSSKTKPFRCKPCQYEAESEEQFVHHIR
VHSAKKFFVEESAEKQAKARESGSSTAEEGDFSKGPIRCDRCGYNTNRYDHYTAHLKHHT
RAGDNERVYKCIICTYTTVSEYHWRKHLRNHFPRKVYTCGKCNYFSDRKNNYVQHVRT
HTGERPYKCELCPYSSSQKTHLTRHMRTHSGEKPFKCDQCSYVASNQHEVTRHARQVHN
GPKPLNCPHCDYKTADRSNFKKHVELHVNPRQFNCPVCDYAASKKCNLQYHFKSKHPTC
PNKTMDVSKVKLKKTKKREADLPDNITNEKTEIEQTKIKGDVAGKKNEKSVKAEKRDVS
KEKKPSNNVSVIQVTTRTRKSVTEVKEMDVHTGSNSEKFSKTKKSKRKLEVDSHSLHGPV
NDEESSTKKKKKVESKSKNNSQEVPKGDSKVEENKKQNTCMKKSTKKKTLKNKSSKKSS
KPPQKEPVEKGSAQMDPPQMGPAPTEAVQKGPVQVEPPPPMEHAQMEGAQIRPAPDEPV
QMEVVQEGPAQKELLPPVEPAQMVGAQIVLAHMELPPPMETAQTEVAQMGPAPMEPAQ
MEVAQVESAPMQVVQKEPVQMELSPPMEVVQKEPVQIELSPPMEVVQKEPVKIELSPPIE
VVQKEPVQMELSPPMGVVQKEPAQREPPPPREPPLHMEPISKKPPLRKDKKEKSNMQSER
ARKEQVLIEVGLVPVKDSWLLKESVSTEDLSPPSPPLPKENLREEASGDQKLLNTGEGNKE
APLQKVGAEEADESLPGLAANINESTHISSSGQNLNTPEGETLNGKHQTDSIVCEMKMDT
DQNTRENLTGINSTVEEPVSPMLPPSAVEEREAVSKTALASPPATMAANESQEIDEDEGIHS
HEGSDLSDNMSEGSDDSGLHGARPVPQESSRKNAKEALAVKAAKGDFVCIFCDRSFRKG
KDYSKHLNRHLVNVYYLEEAAQGQE*
**Amino acid sequence of dCas9 (SEQ ID NO: 21)** 
MAKRNYILGLAIGITSVGYGIIDYETRDVIDAGVRLFKEANVENNEGRRSKRGARRLKRR
RRHRIQRVKKLLFDYNLLTDHSELSGINPYEARVKGLSQKLSEEEFSAALLHLAKRRGVHN
VNEVEEDTGNELSTKEQISRNSKALEEKYVAELQLERLKKDGEVRGSINRFKTSDYVKEA
KQLLKVQKAYHQLDQSFIDTYIDLLETRRTYYEGPGEGSPFGWKDIKEWYEMLMGHCTY
FPEELRSVKYAYNADLYNALNDLNNLVITRDENEKLEYYEKFQIIENVFKQKKKPTLKQIA
 
KEILVNEEDIKGYRVTSTGKPEFTNLKVYHDIKDITARKEIIENAELLDQIAKILTIYQSSEDI
QEELTNLNSELTQEEIEQISNLKGYTGTHNLSLKAINLILDELWHTNDNQIAIFNRLKLVPK
KVDLSQQKEIPTTLVDDFILSPVVKRSFIQSIKVINAIIKKYGLPNDIIIELAREKNSKDAQKM
INEMQKRNRQTNERIEEIIRTTGKENAKYLIEKIKLHDMQEGKCLYSLEAIPLEDLLNNPFN
YEVDHIIPRSVSFDNSFNNKVLVKQEEASKKGNRTPFQYLSSSDSKISYETFKKHILNLAKG
KGRISKTKKEYLLEERDINRFSVQKDFINRNLVDTRYATRGLMNLLRSYFRVNNLDVKVKS
INGGFTSFLRRKWKFKKERNKGYKHHAEDALIIANADFIFKEWKKLDKAKKVMENQMFE
EKQAESMPEIETEQEYKEIFITPHQIKHIKDFKDYKYSHRVDKKPNRKLINDTLYSTRKDDK
GNTLIVNNLNGLYDKDNDKLKKLINKSPEKLLMYHHDPQTYQKLKLIMEQYGDEKNPLY
KYYEETGNYLTKYSKKDNGPVIKKIKYYGNKLNAHLDITDDYPNSRNKVVKLSLKPYRF
DVYLDNGVYKFVTVKNLDVIKKENYYEVNSKCYEEAKKLKKISNQAEFIASFYKNDLIKI
NGELYRVIGVNNDLLNRIEVNMIDITYREYLENMNDKRPPHIIKTIASKTQSIKKYSTDILG
NLYEVKSKKHPQIIKKG
**Amino acid sequence of dspCas9 (SEQ ID NO: 22)** 
MDKKYSIGLAIGTNSVGWAVITDEYKVPSKKFKVLGNTDRHSIKKNLIGALLFDSGETAEA
TRLKRTARRRYTRRKNRICYLQEIFSNEMAKVDDSFFHRLEESFLVEEDKKHERHPIFGNIV
DEVAYHEKYPTIYHLRKKLVDSTDKADLRLIYLALAHMIKFRGHFLIEGDLNPDNSDVDK
LFIQLVQTYNQLFEENPINASGVDAKAILSARLSKSRRLENLIAQLPGEKKNGLFGNLIALS
LGLTPNFKSNFDLAEDAKLQLSKDTYDDDLDNLLAQIGDQYADLFLAAKNLSDAILLSDIL
RVNTEITKAPLSASMIKRYDEHHQDLTLLKALVRQQLPEKYKEIFFDQSKNGYAGYIDGGA
SQEEFYKFIKPILEKMDGTEELLVKLNREDLLRKQRTFDNGSIPHQIHLGELHAILRRQEDF
YPFLKDNREKIEKILTFRIPYYVGPLARGNSRFAWMTRKSEETITPWNFEEVVDKGASAQS
FIERMTNFDKNLPNEKVLPKHSLLYEYFTVYNELTKVKYVTEGMRKPAFLSGEQKKAIVD
LLFKTNRKVTVKQLKEDYFKKIECFDSVEISGVEDRFNASLGTYHDLLKIIKDKDFLDNEE
NEDILEDIVLTLTLFEDREMIEERLKTYAHLFDDKVMKQLKRRRYTGWGRLSRKLINGIRD
KQSGKTILDFLKSDGFANRNFMQLIHDDSLTFKEDIQKAQVSGQGDSLHEHIANLAGSPAI
KKGILQTVKVVDELVKVMGRHKPENIVIEMARENQTTQKGQKNSRERMKRIEEGIKELGS
QILKEHPVENTQLQNEKLYLYYLQNGRDMYVDQELDINRLSDYDVDAIVPQSFLKDDSID
NKVLTRSDKNRGKSDNVPSEEVVKKMKNYWRQLLNAKLITQRKFDNLTKAERGGLSEL
DKAGFIKRQLVETRQITKHVAQILDSRMNTKYDENDKLIREVKVITLKSKLVSDFRKDFQF
YKVREINNYHHAHDAYLNAVVGTALIKKYPKLESEFVYGDYKVYDVRKMIAKSEQEIGK
ATAKYFFYSNIMNFFKTEITLANGEIRKRPLIETNGETGEIVWDKGRDFATVRKVLSMPQV
NIVKKTEVQTGGFSKESILPKRNSDKLIARKKDWDPKKYGGFDSPTVAYSVLVVAKVEKG
KSKKLKSVKELLGITIMERSSFEKNPIDFLEAKGYKEVKKDLIIKLPKYSLFELENGRKRML
ASAGELQKGNELALPSKYVNFLYLASHYEKLKGSPEDNEQKQLFVEQHKHYLDEIIEQISE
FSKRVILADANLDKVLSAYNKHRDKPIREQAENIIHLFTLTNLGAPAAFKYFDTTIDRKRYT
STKEVLDATLIHQSITGLYETRIDLSQLGGD
**Amino acid sequence of dCas9-Krab (SEQ ID NO: 23)** 
MKRTADGSEFESPKKKRKVMDKKYSIGLAIGTNSVGWAVITDEYKVPSKKFKVLGNTDR
HSIKKNLIGALLFDSGETAEATRLKRTARRRYTRRKNRICYLQEIFSNEMAKVDDSFFHRLE
ESFLVEEDKKHERHPIFGNIVDEVAYHEKYPTIYHLRKKLVDSTDKADLRLIYLALAHMIK
FRGHFLIEGDLNPDNSDVDKLFIQLVQTYNQLFEENPINASGVDAKAILSARLSKSRRLENL
 
IAQLPGEKKNGLFGNLIALSLGLTPNFKSNFDLAEDAKLQLSKDTYDDDLDNLLAQIGDQ
YADLFLAAKNLSDAILLSDILRVNTEITKAPLSASMIKRYDEHHQDLTLLKALVRQQLPEK
YKEIFFDQSKNGYAGYIDGGASQEEFYKFIKPILEKMDGTEELLVKLNREDLLRKQRTFDN
GSIPHQIHLGELHAILRRQEDFYPFLKDNREKIEKILTFRIPYYVGPLARGNSRFAWMTRKS
EETITPWNFEEVVDKGASAQSFIERMTNFDKNLPNEKVLPKHSLLYEYFTVYNELTKVKY
VTEGMRKPAFLSGEQKKAIVDLLFKTNRKVTVKQLKEDYFKKIECFDSVEISGVEDRFNA
SLGTYHDLLKIIKDKDFLDNEENEDILEDIVLTLTLFEDREMIEERLKTYAHLFDDKVMKQL
KRRRYTGWGRLSRKLINGIRDKQSGKTILDFLKSDGFANRNFMQLIHDDSLTFKEDIQKAQ
VSGQGDSLHEHIANLAGSPAIKKGILQTVKVVDELVKVMGRHKPENIVIEMARENQTTQK
GQKNSRERMKRIEEGIKELGSQILKEHPVENTQLQNEKLYLYYLQNGRDMYVDQELDINR
LSDYDVDAIVPQSFLKDDSIDNKVLTRSDKNRGKSDNVPSEEVVKKMKNYWRQLLNAKL
ITQRKFDNLTKAERGGLSELDKAGFIKRQLVETRQITKHVAQILDSRMNTKYDENDKLIRE
VKVITLKSKLVSDFRKDFQFYKVREINNYHHAHDAYLNAVVGTALIKKYPKLESEFVYGD
YKVYDVRKMIAKSEQEIGKATAKYFFYSNIMNFFKTEITLANGEIRKRPLIETNGETGEIVW
DKGRDFATVRKVLSMPQVNIVKKTEVQTGGFSKESILPKRNSDKLIARKKDWDPKKYGG
FDSPTVAYSVLVVAKVEKGKSKKLKSVKELLGITIMERSSFEKNPIDFLEAKGYKEVKKDLI
IKLPKYSLFELENGRKRMLASAGELQKGNELALPSKYVNFLYLASHYEKLKGSPEDNEQK
QLFVEQHKHYLDEIIEQISEFSKRVILADANLDKVLSAYNKHRDKPIREQAENIIHLFTLTNL
GAPAAFKYFDTTIDRKRYTSTKEVLDATLIHQSITGLYETRIDLSQLGGDAKRTADGSEFES
PKKKRKVAAADYKDDMGRVTFEDVTVNFTQGEWQRLNPEQRNLYRDVMLENYSNLVSV
GQGETTKPDVILRLEQGKEPWLEEEEVLGSGRAEKNGDIGGQIWKPKDVKESLADYKDD
DDK
**Amino acid sequence of dCas9-3xKrab (SEQ ID NO: 24)** 
MPKKKRKVGIHGVPAAKRNYILGLAIGITSVGYGIIDYETRDVIDAGVRLFKEANVENNEG
RRSKRGARRLKRRRRHRIQRVKKLLFDYNLLTDHSELSGINPYEARVKGLSQKLSEEEFSA
ALLHLAKRRGVHNVNEVEEDTGNELSTKEQISRNSKALEEKYVAELQLERLKKDGEVRG
SINRFKTSDYVKEAKQLLKVQKAYHQLDQSFIDTYIDLLETRRTYYEGPGEGSPFGWKDIK
EWYEMLMGHCTYFPEELRSVKYAYNADLYNALNDLNNLVITRDENEKLEYYEKFQIIENV
FKQKKKPTLKQIAKEILVNEEDIKGYRVTSTGKPEFTNLKVYHDIKDITARKEIIENAELLD
QIAKILTIYQSSEDIQEELTNLNSELTQEEIEQISNLKGYTGTHNLSLKAINLILDELWHTNDN
QIAIFNRLKLVPKKVDLSQQKEIPTTLVDDFILSPVVKRSFIQSIKVINAIIKKYGLPNDIIIEL
AREKNSKDAQKMINEMQKRNRQTNERIEEIIRTTGKENAKYLIEKIKLHDMQEGKCLYSL
EAIPLEDLLNNPFNYEVDHIIPRSVSFDNSFNNKVLVKQEEASKKGNRTPFQYLSSSDSKIS
YETFKKHILNLAKGKGRISKTKKEYLLEERDINRFSVQKDFINRNLVDTRYATRGLMNLLR
SYFRVNNLDVKVKSINGGFTSFLRRKWKFKKERNKGYKHHAEDALIIANADFIFKEWKKL
DKAKKVMENQMFEEKQAESMPEIETEQEYKEIFITPHQIKHIKDFKDYKYSHRVDKKPNR
KLINDTLYSTRKDDKGNTLIVNNLNGLYDKDNDKLKKLINKSPEKLLMYHHDPQTYQKL
KLIMEQYGDEKNPLYKYYEETGNYLTKYSKKDNGPVIKKIKYYGNKLNAHLDITDDYPN
SRNKVVKLSLKPYRFDVYLDNGVYKFVTVKNLDVIKKENYYEVNSKCYEEAKKLKKISN
QAEFIASFYKNDLIKINGELYRVIGVNNDLLNRIEVNMIDITYREYLENMNDKRPPHIIKTIA
SKTQSIKKYSTDILGNLYEVKSKKHPQIIKKGKRPAATKKAGQAKKKKGSGSGGGGSGGG
GSMGRVTFEDVTVNFTQGEWQRLNPEQRNLYRDVMLENYSNLVSVGQGETTKPDVILRL
EQGKEPWLEEEEVLGSGRAEKNGDIGGQIWKPKDVKESLGGGGSMGRVTFEDVTVNFTQ
 
GEWQRLNPEQRNLYRDVMLENYSNLVSVGQGETTKPDVILRLEQGKEPWLEEEEVLGSG
RAEKNGDIGGQIWKPKDVKESLGGGGSMGRVTFEDVTVNFTQGEWQRLNPEQRNLYRD
VMLENYSNLVSVGQGETTKPDVILRLEQGKEPWLEEEEVLGSGRAEKNGDIGGQIWKPK
DVKESLAAADYKDDDDK*
**Amino acid sequence of N-dCas9 (SEQ ID NO: 25)** 
MPKKKRKVGIHGVPAAKRNYILGLAIGITSVGYGIIDYETRDVIDAGVRLFKEANVENNEG
RRSKRGARRLKRRRRHRIQRVKKLLFDYNLLTDHSELSGINPYEARVKGLSQKLSEEEFSA
ALLHLAKRRGVHNVNEVEEDTGNELSTKEQISRNSKALEEKYVAELQLERLKKDGEVRG
SINRFKTSDYVKEAKQLLKVQKAYHQLDQSFIDTYIDLLETRRTYYEGPGEGSPFGWKDIK
EWYEMLMGHCTYFPEELRSVKYAYNADLYNALNDLNNLVITRDENEKLEYYEKFQIIENV
FKQKKKPTLKQIAKEILVNEEDIKGYRVTSTGKPEFTNLKVYHDIKDITARKEIIENAELLD
QIAKILTIYQSSEDIQEELTNLNSELTQEEIEQISNLKGYTGTHNLSLKAINLILDELWHTNDN
QIAIFNRLKLVPKKVDLSQQKEIPTTLVDDFILSPVVKRSFIQSIKVINAIIKKYGLPNDIIIEL
AREKNSKDAQKMINEMQKRNRQTNERIEEIIRTTGKENAKYLIEKIKLHDMQEGKCLYSL
EAIPLEDLLNNPFNYEVDHIIPRSVSFDNSFNNKVLVKQEEASKKGNRTPFQYLSSSDSKIS
YETFKKHILNLAKGKGRISKTKKEYLLEERDINRFSVQKDFINRNLVDTRYATRGLMNLLR
SYFRVNNLDVKVKSINGGFTSFLRRKWKFKKERNKGYKHHAEDALIIANADFIFKEWKKL
DKAKKVMENQMFEEKQAESMPEIETEQEYKEIFITPHQIKHIKDFKDYKYSHRVDKKPNR
KLINDTLYSTRKDDKGNTLIVNNLNGLYDKDNDKLKKLINKSPEKLLMYHHDPQTYQKL
KLIMEQYGDEKNPLYKYYEETGNYLTKYSKKDNGPVIKKIKYYGNKLNAHLDITDDYPN
SRNKVVKLSLKPYRFDVYLDNGVYKFVTVKNLDVIKKENYYEVNSKCYEEAKKLKKISN
QAEFIASFYKNDLIKINGELYRVIGVNNDLLNRIEVNMIDITYREYLENMNDKRPPHIIKTIA
SKTQSIKKYSTDILGNLYEVKSKKHPQIIKKGKRPAATKKAGQAKKKKGSMATQVMGQSS
GGGGLFTSSGNIGMALPNDMYDLHDLSKAELAAPQLIMLANVALTGEVNGSCCDYLVGE
ERQMAELMPVGDNNFSDSEEGEGLEESADIKGEPHGLENMELRGGGGSMATQVMGQSS
GGGGLFTSSGNIGMALPNDMYDLHDLSKAELAAPQLIMLANVALTGEVNGSCCDYLVGE
ERQMAELMPVGDNNFSDSEEGEGLEESADIKGEPHGLENMELRGGGGSMATQVMGQSS
GGGGLFTSSGNIGMALPNDMYDLHDLSKAELAAPQLIMLANVALTGEVNGSCCDYLVGE
ERQMAELMPVGDNNFSDSEEGEGLEESADIKGEPHGLENMELRGSGGGGSGGGGSAAA
DYKDDDDK*
**Amino acid sequence of 3xN-dCas9 (SEQ ID NO: 26)** 
MPKKKRKVGIHGVPAKKKKGSMATQVMGQSSGGGGLFTSSGNIGMALPNDMYDLHDLS
KAELAAPQLIMLANVALTGEVNGSCCDYLVGEERQMAELMPVGDNNFSDSEEGEGLEES
ADIKGEPHGLENMELRGGGGSMATQVMGQSSGGGGLFTSSGNIGMALPNDMYDLHDLS
KAELAAPQLIMLANVALTGEVNGSCCDYLVGEERQMAELMPVGDNNFSDSEEGEGLEES
ADIKGEPHGLENMELRGGGGSMATQVMGQSSGGGGLFTSSGNIGMALPNDMYDLHDLS
KAELAAPQLIMLANVALTGEVNGSCCDYLVGEERQMAELMPVGDNNFSDSEEGEGLEES
ADIKGEPHGLENMELRGSGGGGSGGGGSAKRNYILGLAIGITSVGYGIIDYETRDVIDAGV
RLFKEANVENNEGRRSKRGARRLKRRRRHRIQRVKKLLFDYNLLTDHSELSGINPYEARV
KGLSQKLSEEEFSAALLHLAKRRGVHNVNEVEEDTGNELSTKEQISRNSKALEEKYVAEL
QLERLKKDGEVRGSINRFKTSDYVKEAKQLLKVQKAYHQLDQSFIDTYIDLLETRRTYYE
GPGEGSPFGWKDIKEWYEMLMGHCTYFPEELRSVKYAYNADLYNALNDLNNLVITRDEN
 
EKLEYYEKFQIIENVFKQKKKPTLKQIAKEILVNEEDIKGYRVTSTGKPEFTNLKVYHDIKD
ITARKEIIENAELLDQIAKILTIYQSSEDIQEELTNLNSELTQEEIEQISNLKGYTGTHNLSLKA
INLILDELWHTNDNQIAIFNRLKLVPKKVDLSQQKEIPTTLVDDFILSPVVKRSFIQSIKVINA
IIKKYGLPNDIIIELAREKNSKDAQKMINEMQKRNRQTNERIEEIIRTTGKENAKYLIEKIKL
HDMQEGKCLYSLEAIPLEDLLNNPFNYEVDHIIPRSVSFDNSFNNKVLVKQEEASKKGNRT
PFQYLSSSDSKISYETFKKHILNLAKGKGRISKTKKEYLLEERDINRFSVQKDFINRNLVDT
RYATRGLMNLLRSYFRVNNLDVKVKSINGGFTSFLRRKWKFKKERNKGYKHHAEDALIIA
NADFIFKEWKKLDKAKKVMENQMFEEKQAESMPEIETEQEYKEIFITPHQIKHIKDFKDY
KYSHRVDKKPNRKLINDTLYSTRKDDKGNTLIVNNLNGLYDKDNDKLKKLINKSPEKLL
MYHHDPQTYQKLKLIMEQYGDEKNPLYKYYEETGNYLTKYSKKDNGPVIKKIKYYGNK
LNAHLDITDDYPNSRNKVVKLSLKPYRFDVYLDNGVYKFVTVKNLDVIKKENYYEVNSK
CYEEAKKLKKISNQAEFIASFYKNDLIKINGELYRVIGVNNDLLNRIEVNMIDITYREYLEN
MNDKRPPHIIKTIASKTQSIKKYSTDILGNLYEVKSKKHPQIIKKGKRPAATKKAGQAKKK
KGSAAADYKDDDDK*
**Amino acid sequence of 3xN-dCas9-3xC (SEQ ID NO: 27)** 
MPKKKRKVGIHGVPAKKKKGSMATQVMGQSSGGGGLFTSSGNIGMALPNDMYDLHDLS
KAELAAPQLIMLANVALTGEVNGSCCDYLVGEERQMAELMPVGDNNFSDSEEGEGLEES
ADIKGEPHGLENMELRGGGGSMATQVMGQSSGGGGLFTSSGNIGMALPNDMYDLHDLS
KAELAAPQLIMLANVALTGEVNGSCCDYLVGEERQMAELMPVGDNNFSDSEEGEGLEES
ADIKGEPHGLENMELRGGGGSMATQVMGQSSGGGGLFTSSGNIGMALPNDMYDLHDLS
KAELAAPQLIMLANVALTGEVNGSCCDYLVGEERQMAELMPVGDNNFSDSEEGEGLEES
ADIKGEPHGLENMELRGSGGGGSGGGGSAKRNYILGLAIGITSVGYGIIDYETRDVIDAGV
RLFKEANVENNEGRRSKRGARRLKRRRRHRIQRVKKLLFDYNLLTDHSELSGINPYEARV
KGLSQKLSEEEFSAALLHLAKRRGVHNVNEVEEDTGNELSTKEQISRNSKALEEKYVAEL
QLERLKKDGEVRGSINRFKTSDYVKEAKQLLKVQKAYHQLDQSFIDTYIDLLETRRTYYE
GPGEGSPFGWKDIKEWYEMLMGHCTYFPEELRSVKYAYNADLYNALNDLNNLVITRDEN
EKLEYYEKFQIIENVFKQKKKPTLKQIAKEILVNEEDIKGYRVTSTGKPEFTNLKVYHDIKD
ITARKEIIENAELLDQIAKILTIYQSSEDIQEELTNLNSELTQEEIEQISNLKGYTGTHNLSLKA
INLILDELWHTNDNQIAIFNRLKLVPKKVDLSQQKEIPTTLVDDFILSPVVKRSFIQSIKVINA
IIKKYGLPNDIIIELAREKNSKDAQKMINEMQKRNRQTNERIEEIIRTTGKENAKYLIEKIKL
HDMQEGKCLYSLEAIPLEDLLNNPFNYEVDHIIPRSVSFDNSFNNKVLVKQEEASKKGNRT
PFQYLSSSDSKISYETFKKHILNLAKGKGRISKTKKEYLLEERDINRFSVQKDFINRNLVDT
RYATRGLMNLLRSYFRVNNLDVKVKSINGGFTSFLRRKWKFKKERNKGYKHHAEDALIIA
NADFIFKEWKKLDKAKKVMENQMFEEKQAESMPEIETEQEYKEIFITPHQIKHIKDFKDY
KYSHRVDKKPNRKLINDTLYSTRKDDKGNTLIVNNLNGLYDKDNDKLKKLINKSPEKLL
MYHHDPQTYQKLKLIMEQYGDEKNPLYKYYEETGNYLTKYSKKDNGPVIKKIKYYGNK
LNAHLDITDDYPNSRNKVVKLSLKPYRFDVYLDNGVYKFVTVKNLDVIKKENYYEVNSK
CYEEAKKLKKISNQAEFIASFYKNDLIKINGELYRVIGVNNDLLNRIEVNMIDITYREYLEN
MNDKRPPHIIKTIASKTQSIKKYSTDILGNLYEVKSKKHPQIIKKGKRPAATKKAGQAKKK
KGSAGSGGGGSGGGGSMDEGIHSHEGSDLSDNMSEGSDDSGLHGARPVPQESSRKNAKE
ALAVKAAKGDFVCIFCDRSFRKGKDYSKHLNRHLVNVYYLEEAAQGQEGGGGSMDEGI
HSHEGSDLSDNMSEGSDDSGLHGARPVPQESSRKNAKEALAVKAAKGDFVCIFCDRSFRK
GKDYSKHLNRHLVNVYYLEEAAQGQEGGGGSMDEGIHSHEGSDLSDNMSEGSDDSGLH
 
GARPVPQESSRKNAKEALAVKAAKGDFVCIFCDRSFRKGKDYSKHLNRHLVNVYYLEEA
AQGQEAAADYKDDDDK*
**Amino acid sequence of Krab (SEQ ID NO: 28)** 
MGRVTFEDVTVNFTQGEWQRLNPEQRNLYRDVMLENYSNLVSVGQGETTKPDVILRLEQ
GKEPWLEEEEVLGSGRAEKNGDIGGQIWKPKDVKESL
**Amino acid sequence of Ctdsp1 (SEQ ID NO: 29)** 
MDSSAVITQISKEEARGPLRGKGDQKSAASQKPRSRGILHSLFCCVCRDDGEALPAHSGAP
LLVEENGAIPKQTPVQYLLPEAKAQDSDKICVVIDLDETLVHSSFKPVNNADFIIPVEIDGV
VHQVYVLKRPHVDEFLQRMGELFECVLFTASLAKYADPVADLLDKWGAFRARLFRESCV
FHRGNYVKDLSRLGRDLRRVLILDNSPASYVFHPDNAVPVASWFDNMSDTELHDLLPFFE
QLSRVDDVYSVLRQPRPGS*
**Nucleotide sequence of Ctdsp1 (SEQ ID NO:30)** 
Atggacagctcggccgtcattactcagatcagcaaggaggaggctcggggcccgctgcggggcaaaggtgaccagaagtcagcagcttc
ccagaagccccgaagccggggcatcctccactcactcttctgctgtgtctgccgggatgatggggaggccctgcctgctcacagcggggcg
cccctgcttgtggaggagaatggcgccatccctaagcagaccccagtccaatacctgctccctgaggccaaggcccaggactcagacaaga
tctgcgtggtcatcgacctggacgagaccctggtgcacagctccttcaagccagtgaacaacgcggacttcatcatccctgtggagattgatg
gggtggtccaccaggtctacgtgttgaagcgtcctcacgtggatgagttcctgcagcgaatgggcgagctctttgaatgtgtgctgttcactgct
agcctcgccaagtacgcagacccagtagctgacctgctggacaaatggggggccttccgggcccggctgtttcgagagtcctgcgtcttcca
ccgggggaactacgtgaaggacctgagccggttgggtcgagacctgcggcgggtgctcatcctggacaattcacctgcctcctatgtcttcc
atccagacaatgctgtaccggtggcctcgtggtttgacaacatgagtgacacagagctccacgacctcctccccttcttcgagcaactcagcc
gtgtggacgacgtgtactcagtgctcaggcagccacggccagggagctag
**Coding sequence of human miRNA-124 (SEQ ID NO:31)**
   5' -cgtgttcacagcggaccttgat-3'
**Coding sequence of human miRNA-124* (SEQ ID NO:32)**
   5'-taaggcacgcggtgaatgccaa-3'
**The full sequence of human miRNA-124 Pre-miRNA (SEQ ID NO:33)** 
atcaagattagaggctctgctctccgtgttcacagcggaccttgatttaatgtcatacaattaaggcacgcggtgaatgccaagagcggagcct
acggctgcacttgaa
**The sequence expressing human miRNA-124 Pri-miRNA (SEQ ID NO:34)** 
Gggtaattaacttggatccacgccgtcatttgaaaactagatttataggcttatgtatgtttttaggcgtgtgctgtaaatggcatggagatatatgc
atatgtatacgcaggcacacgcaccgtctacacttccacggaacagactaattaacagcggctctggcagatgtgtcagagatgagcagaga
caggagctgggcttatgagttatgactctaggggtagagactcagagcggagagagggggatgggcagggagagaagagtggtaatcgc
agtgggtcttatactttccggatcaagattagaggctctgctctccgtgttcacagcggaccttgatttaatgtcatacaattaaggcacgcggtg
aatgccaagagcggagcctacggctgcacttgaaggacaccaaagcatctcagggtcagaaaggggaaaaagcaattgcagggaatttag
ggggtagtaaaaggaacccatctcttgccgcataaatgccccccacccccacccaggactgattctggaagcaacctagtgttcgaaaggga
aaggctcctacttttccattacagccgcggaaatccgcaggcaaatctccgaggagaattttagggaagcttcattgacagctgtctggagagc
agtagttcccgcctgtgcaaatattccagagagttaaatcatttagaaagcactagtt
**Coding sequence of human miRNA-9**
   5'- tctttggttatctagctgtatga-3' (SEQ ID NO:35)
**Coding sequence of human miRNA-9***
   5'- ataaagctagataaccgaaagt-3' (SEQ ID NO:36)
**The full sequence of miRNA-9 Per-miRNA (SEQ ID NO:37)**
   Ggaggcccgtttctctctttggttatctagctgtatgagtgccacagagccgtcataaagctagataaccgaaagtagaaatgattctca
**The sequence expressing human miRNA-9 Pri-miRNA (SEQ ID NO:38)** 
Agcgctccgcgcgggtgccctacgtgagccccgggacgccgtcgaggcaggccggcagcgccggtgccaggacgcacggaacgggg
agcaggggagaaatgcgccgggagggcgagggaggaagggaactgggcgggggctgcgggcctaggtggcgggagtcagcgtgtgc
gtgtgtctgtccatcccctctggctctccgcgtgcgccccaggatccgggcacggcgtccgctcaggctcccgcgctcggcaggcagcagc
acgtggagcccacggcgcggcagcggcactggctaagggaggcccgtttctctctttggttatctagctgtatgagtgccacagagccgtcat
aaagctagataaccgaaagtagaaatgattctcacaacttctgcgtgcgagcgcccgccccaccgaccgccccggcccggcccgcaagag
ccagaggagccgagaggagcccagcgccggcccagcggactccagctcgacggagcggccgcgccccgaccaggtacctgacctcgg
ctctctctctggggacacttgcagtgccagctttcccccagtcctcctctttcctggtcccccacccctatcccccagcgccggagcaggaacc
ccaaagtggagcgggccggggagagggagggggttggggttgcagaggggtcgctggaagggg
**The long promoter of GFAP (SEQ ID NO:39)** 
TAATCCCACCTCCCTCTCTGTGCTGGGACTCACAGAGGGAGACCTCAGGAGGCAGTCT
GTCCATCACATGTCCAAATGCAGAGCATACCCTGGGCTGGGCGCAGTGGCGCACAACT
GTAATTCCAGCACTTTGGGAGGCTGATGTGGAAGGATCACTTGAGCCCAGAAGTTCTA
GACCAGCCTGGGCAACATGGCAAGACCCTATCTCTACAAAAAAAGTTAAAAAATCAGC
CACGTGTGGTGACACACACCTGTAGTCCCAGCTATTCAGGAGGCTGAGGTGAGGGGAT
CACTTAAGGCTGGGAGGTTGAGGCTGCAGTGAGTCGTGGTTGCGCCACTGCACTCCA
GCCTGGGCAACAGTGAGACCCTGTCTCAAAAGACAAAAAAAAAAAAAAAAAAAAAA
GAACATATCCTGGTGTGGAGTAGGGGACGCTGCTCTGACAGAGGCTCGGGGGCCTGA
GCTGGCTCTGTGAGCTGGGGAGGAGGCAGACAGCCAGGCCTTGTCTGCAAGCAGACC
TGGCAGCATTGGGCTGGCCGCCCCCCAGGGCCTCCTCTTCATGCCCAGTGAATGACTC
ACCTTGGCACAGACACAATGTTCGGGGTGGGCACAGTGCCTGCTTCCCGCCGCACCCC
AGCCCCCCTCAAATGCCTTCCGAGAAGCCCATTGAGCAGGGGGCTTGCATTGCACCCC
AGCCTGACAGCCTGGCATCTTGGGATAAAAGCAGCACAGCCCCCTAGGGGCTGCCCTT
GCTGTGTGGCGCCACCGGCGGTGGAGAACAAGGCTCTATTCAGCCTGTGCCCAGGAA
AGGGGATCAGGGGATGCCCAGGCATGGACAGTGGGTGGCAGGGGGGGAGAGGAGGG
CTGTCTGCTTCCCAGAAGTCCAAGGACACAAATGGGTGAGGGGACTGGGCAGGGTTC
TGACCCTGTGGGACCAGAGTGGAGGGCGTAGATGGACCTGAAGTCTCCAGGGACAAC
AGGGCCCAGGTCTCAGGCTCCTAGTTGGGCCCAGTGGCTCCAGCGTTTCCAAACCCAT
CCATCCCCAGAGGTTCTTCCCATCTCTCCAGGCTGATGTGTGGGAACTCGAGGAAATA
AATCTCCAGTGGGAGACGGAGGGGTGGCCAGGGAAACGGGGCGCTGCAGGAATAAA
GACGAGCCAGCACAGCCAGCTCATGTGTAACGGCTTTGTGGAGCTGTCAAGGCCTGGT
CTCTGGGAGAGAGGCACAGGGAGGCCAGACAAGGAAGGGGTGACCTGGAGGGACAG
ATCCAGGGGCTAAAGTCCTGATAAGGCAAGAGAGTGCCGGCCCCCTCTTGCCCTATCA
 
GGACCTCCACTGCCACATAGAGGCCATGATTGACCCTTAGACAAAGGGCTGGTGTCCA
ATCCCAGCCCCCAGCCCCAGAACTCCAGGGAATGAATGGGCAGAGAGCAGGAATGTG
GGACATCTGTGTTCAAGGGAAGGACTCCAGGAGTCTGCTGGGAATGAGGCCTAGTAG
GAAATGAGGTGGCCCTTGAGGGTACAGAACAGGTTCATTCTTCGCCAAATTCCCAGCA
CCTTGCAGGCACTTACAGCTGAGTGAGATAATGCCTGGGTTATGAAATCAAAAAGTTG
GAAAGCAGGTCAGAGGTCATCTGGTACAGCCCTTCCTTCCCTTTTTTTTTTTTTTTTTTG
TGAGACAAGGTCTCTCTCTGTTGCCCAGGCTGGAGTGGCGCAAACACAGCTCACTGC
AGCCTCAACCTACTGGGCTCAAGCAATCCTCCAGCCTCAGCCTCCCAAAGTGCTGGGA
TTACAAGCATGAGCCACCCCACTCAGCCCTTTCCTTCCTTTTTAATTGATGCATAATAAT
TGTAAGTATTCATCATGGTCCAACCAACCCTTTCTTGACCCACCTTCCTAGAGAGAGGG
TCCTCTTGCTTCAGCGGTCAGGGCCCCAGACCCATGGTCTGGCTCCAGGTACCACCTG
CCTCATGCAGGAGTTGGCGTGCCCAGGAAGCTCTGCCTCTGGGCACAGTGACCTCAGT
GGGGTGAGGGGAGCTCTCCCCATAGCTGGGCTGCGGCCCAACCCCACCCCCTCAGGCT
ATGCCAGGGGGTGTTGCCAGGGGCACCCGGGCATCGCCAGTCTAGCCCACTCCTTCAT
AAAGCCCTCGCATCCCAGGAGCGAGCAGAGCCAGAGCAGGTTGGAGAGGAGACGCA
TCACCTCCGCTGCTCGCGG
**The short promoter of GFAP (SEQ ID NO: 40)**

AACATATCCTGGTGTGGAGTAGGGGACGCTGCTCTGACAGAGGCTCGGGGGCCTGAG
CTGGCTCTGTGAGCTGGGGAGGAGGCAGACAGCCAGGCCTTGTCTGCAAGCAGACCT
GGCAGCATTGGGCTGGCCGCCCCCCAGGGCCTCCTCTTCATGCCCAGTGAATGACTCA
CCTTGGCACAGACACAATGTTCGGGGTGGGCACAGTGCCTGCTTCCCGCCGCACCCCA
GCCCCCCTCAAATGCCTTCCGAGAAGCCCATTGAGCAGGGGGCTTGCATTGCACCCCA
GCCTGACAGCCTGGCATCTTGGGATAAAAGCAGCACAGCCCCCTAGGGGCTGCCCTTG
CTGTGTGGCGCCACCGGCGGTGGAGAACAAGGCTCTATTCAGCCTGTGCCCAGGAAA
GGGGATCAGGGGATGCCCAGGCATGGACAGTGGGTGGCAGGGGGGGAGAGGAGGGC
TGTCTGCTTCCCAGAAGTCCAAGGACACAAATGGGTGAGGGGAGAGCTCTCCCCATAG
CTGGGCTGCGGCCCAaccccaccccctcaggctatgccagggggtgttgccaggggcacccgggcatcgccagtctagccc
actccttcataaagccctcgcatcccaggagcgagcagagccagagcaggttggagaggagacgcatcacctccgctgctcgc

## Claims

1. A method for trans-differentiating non-neuronal cells to functional neurons in a subject, comprising administering to the subject an active substance capable of reducing the binding of REST to RE1/NRSE elements, or reducing the amount or activity of REST.

2. A method for preventing and/or treating diseases associated with neuronal dysfunction or death in a subject in need, comprising administering a therapeutically effective dose of an active substance capable of reducing the binding of REST to the RE1/NRSE element, or reducing the amount or activity of REST, to the areas affected by the disease in the subject, in order to transdifferentiate non-neuronal cells in the affected areas into functional neurons.

3. The method of claim 1 or 2, wherein the said functional neurons include dopamine neurons, retinal ganglion cells, photoreceptor cells and cochlear spiral ganglion cells, GABA neurons, 5-HT neurons, glutamatergic neurons, ChAT neurons, NE neurons, motor neurons, spinal neurons, spinal motor neurons, spinal sensory neurons, bipolar cells, horizontal cells, amacrine cells, pyramidal neurons, interneurons neurons, medium spiny neurons (MSNs), Purkinje cells, granule cells, olfactory sensory neurons, periglomerular cells, or any combination thereof.

4. The method of any one of preceding claims, wherein the functional neurons express the NeuN gene.

5. The method of any one of preceding claims, wherein the functional neurons have axons.

6. The method of any one of preceding claim, wherein the functional neurons comprise dopamine neurons, retinal ganglion cells, photoreceptor cells or cochlear spiral ganglion cells.

7. The method of claim 6, wherein the dopamine neurons express one or more markers selected from tyrosine hydroxylase (TH), FoxA2, Nurr1, Pitx3, Vmat2 and DAT.

8. The method of claim 6, wherein the dopamine neurons express NeuN, TH, and DAT.

9. The method of claim 6, wherein the retinal ganglion cells express one or more markers selected from RBPMS, Pax6, Brn3a, Brn3b, Brn3c, and Map2.

10. The method of claim 6, wherein the photoreceptor cells express one or more markers selected from Rhodopsin, mCAR, m-opsin and S-opsin.

11. The method of 6, wherein the cochlear spiral ganglion cells express one or more markers selected from NeuN, Prox1, Tuj-1 and Map2.

12. The method of any one of the preceding claims, wherein the non-neuronal cells comprise glial cells, fibroblasts, stem cells, neural precursor cells, or neural stem cells.

13. The method of claim 12, wherein the glial cells are selected from astrocytes, oligodendrocytes, ependymal cells, Schwann cells, NG2 cells, satellite cells, Müller glial cells, inner ear glial cells, and any combination thereof.

14. The method of claim 13, wherein the glial cells are selected from astrocytes, Müller glia cells and cochlear glia cells.

15. The method of any one of claims 12-14, wherein the glial cells are located in the brain, spinal cord, eye or ear.

16. The method of claim 15, wherein the glial cells are located in the striatum, substantia nigra, ventral tegmental area of the midbrain, medulla oblongata, hypothalamus, dorsal midbrain, or cerebral cortex of the brain.

17. The method of any one of the preceding claims, wherein the active substance is administered locally to glial cells in one or more of the following locations in the subject: 1) glial cells in the striatum; ii) glial cells in the substantia nigra of the brain; iii) glial cells in the retina; iv) glial cells in the inner ear; v) glial cells in the spinal cord; vi) glial cells in the prefrontal cortex; vii) glial cells in motor cortex ; viii) glial cells in the hypothalamus; and ix) glial cells in the ventral tegmental area (VTA).

18. The method of any one of the preceding claims, wherein said glial cells comprise astrocytes and said functional neurons comprise dopamine neurons.

19. The method of claim 18, wherein the astrocytes are located in the striatum and/or the substantia nigra.

20. The method according to any one of claims 1-17, wherein said glial cells comprise Müller glial cells, and said functional neurons comprise retinal ganglion cells (RGCs) and/or photoreceptor cells.

21. The method of claim 20, wherein the Müller glial cells are in the retina or vitreous cavity.

22. The method of any one of claims 1-17, wherein the glial cells comprise cochlear glial cells, and the functional neurons comprise cochlear spiral ganglion cells.

23. The method of claim 22, wherein the cochlear glial cells are located in the inner ear.

24. The method of any one of the preceding claims, wherein the trans-differentiation efficiency of the glial cells into functional neurons achieved after the administration of the active substance is at least 1%, or at least 10%, 20%, 30%, 40% or 50%.

25. The method of any one of the preceding claims, wherein the active substance comprises a RE1/NRSE element blocker, which could bind to the RE1/NRSE element to block the binding of REST and the RE1/NRSE element.

26. The method of claim 25, wherein the RE1/NRSE element blocker comprises a small molecule compound, a nucleic acid, or a nucleic acid analog that competes with REST for binding to RE1.

27. The method of claim 25, wherein said RE1/NRSE element blocker comprises a protein that competes with REST for binding to RE1 or a nucleic acid encoding said protein.

28. The method of claim 27, wherein the protein that competes with REST for binding to RE1comprises a REST variant.

29. The method of claim 28, wherein the REST variant comprises the DNA binding domain of REST but lacks the N-terminal and/or C-terminal repression domain of REST.

30. The method of claim 28 or 29, wherein the REST variant comprises amino acids from 155 to 420 of REST, but lacks the N-terminal and/or C-terminal inhibitory domain of REST.

31. The method of any one of claims 28-30, wherein the REST variant comprises the amino acid sequence of SEQ ID NO: 1, 3, 5 or 9, or comprises the amino acid sequence of at least 70%, 60%, or 50% identity percentage with anyone thereof.

32. The method according to claim 27, wherein said RE1/NRSE element blocker comprises a nucleic acid encoding the REST variant as claimed in any one of claims 28-31, said nucleic acid encoding the REST variant comprises the sequence of SEQ ID NO: 2, 4, 6 or 10, or comprises the sequence of at least 70%, 60%, or 50% identity percentage with anyone thereof.

33. The method of claim 32, wherein the nucleic acid encoding the REST variant is codon-optimized, alternatively comprises the nucleotide sequence of SEQ ID NO: 15, or at least 70%, 60% or 50 identity percentage with SEQ ID NO: 15.

34. The method of claims 28-31, wherein said REST variant further comprises an activation domain fused to the DNA binding domain of REST.

35. The method according to claim 34, wherein the activation domain comprises an epigenetic modification protein or a gene activation regulatory element, optionally, the activation domain comprises VP64, P65-HSF1, VP16, RTA, Suntag, P300, CBP or any combination thereof, optionally, the activation domain comprises VP64 or P65-HSF1.

36. The method of any one of claims 28-35, wherein the REST variant is fused to one or more nuclear localization signal sequences.

37. The method of claim 36, wherein at least one of said nuclear localization signal sequences is fused to the N-terminus of said REST variant.

38. The method of claim 36 or 37, wherein at least one of said nuclear localization signal sequences is fused to the C-terminus of said REST variant.

39. The method of any one of claims 36-38, wherein at least one of said nuclear localization signal sequences is fused to the N-terminus and C-terminus of said REST variant, respectively.

40. The method according to any one of claims 36-39, wherein the nuclear localization signal sequence comprises the amino acid sequence selected from SEQ ID NOs: 13, 41-58.

41. The method of any one of the preceding claims, wherein the subject is a human or an animal.

42. The method of claim 41, wherein the animal is a non-human primate, rat or mouse.

43. The method according to claim 2, wherein the diseases associated with neuronal dysfunction or death are selected from: Parkinson's disease, Alzheimer's disease, stroke, schizophrenia, Huntington's disease, depression, motor neuron disease, amyotrophic lateral sclerosis, spinal muscular atrophy, Pick disease, sleep disorders, epilepsy, ataxia, visual impairment due to RGC death, glaucoma, age-related RGC lesions, optic nerve injury, retinal ischemia or hemorrhage, Leber hereditary optic neuropathy, degeneration or death of photoreceptor cells due to injury or degeneration, macular degeneration, retinitis pigmentosa, diabetes-related blindness, night blindness, color blindness, hereditary blindness, congenital amaurosis, deafness or hearing loss due to spiral ganglion cell death, and any combination thereof.

44. A REST variant comprising the DNA binding domain of REST but lacking the N-terminal and/or C-terminal repression domain of REST.

45. The REST variant of claim 44, comprising amino acids from 155 to 420 of REST, but lacking the N-terminal and/or C-terminal repression domain of REST.

46. The REST variant of claim 44 or 45, which comprises the amino acid sequence of SEQ ID NO: 1, 3, 5 or 9, or comprises a sequence having at least 70%, 60%, or 50% identity percentage with anyone thereof.

47. The REST variant of any one of claims 44-46, which comprises a nucleotide sequence of SEQ ID NO: 2, 4, 6 or 10, or comprises a nucleotide sequence of at least 70%, 60%, or 50% identity percentage with anyone thereof.

48. The REST variant of any one of claims 44-47, further comprising an activation domain fused to the DNA binding domain of said REST.

49. The REST variant of claim 48, wherein the activation domain comprises an epigenetic modification protein or a gene activation regulatory element, optionally, the activation domain comprises VP64, P65-HSF1, VP16, RTA, Suntag, P300, CBP or any combination thereof, optionally, the activation domain includes VP64 or P65-HSF1.

50. The REST variant of any one of claims 44-49, which fused to one or more nuclear localization signal sequences.

51. The REST variant of claim 50, wherein at least one of said nuclear localization signal sequences is fused to the N-terminus of said REST variant.

52. The REST variant of claim 50 or 51, wherein at least one of said nuclear localization signal sequences is fused to the C-terminus of said REST variant.

53. The REST variant of any one of claims 50-52, wherein at least one of said nuclear localization signal sequences is fused to the N-terminus and -C-terminus of said REST variant, respectively.

54. The REST variant of any one of claims 50-53, wherein the nuclear localization signal sequence comprises the amino acid sequence shown in SEQ ID NO:13.

55. A polynucleotide comprising a nucleic acid sequence encoding the REST variant according to any one of claims 44-54.

56. An expression vector comprising the polynucleotide of claim 55, and optionally further comprising a promoter operably linked to the polynucleotide.

57. The expression vector of claim 56, wherein the promoter is a glial cell-specific promoter.

58. The expression vector according to claim 57, the glial cell-specific promoter is an astrocyte-specific promoter or a Müller glial cell (MG) cell-specific promoter.

59. The expression vector of claim 57 or 58, wherein the glial cell-specific promoter is selected from GFAP promoter, ALDH1L1 promoter, EAAT1/GLAST promoter, glutamine synthetase promoter, S100β promoter EAAT2 /GLT-1 promoter and Rlbp1 promoter, preferably selected from GFAP promoter.

60. The expression vector of any one of claims 57-59, wherein the glial cell-specific promoter is a cochlear glial cell-specific promoter.

61. The expression vector of claim 60, the cochlear glial cell-specific promoter is selected from GFAP promoter, ALDH1L1 promoter, EAAT1/GLAST promoter and Plp1 promoter.

62. A pharmaceutical composition comprising the REST variant of any one of claims 44-54 or the polynucleotide of claim 55 or the expression vector of any one of claims 56-60, and pharmaceutically acceptable carrier.

63. The pharmaceutical composition of claim 62, further comprising a carrier for delivering the polynucleotide, wherein the carrier comprises a viral vector, liposome, nanoparticle, exosome, or virus-like particles.

64. The pharmaceutical composition of claim 63, wherein the viral vector comprises recombinant adeno-associated viral vector (rAAV), adeno-associated viral (AAV) vector, adenoviral vector, lentiviral vector, retroviral vector, poxvirus vector, herpes virus vector, SV40 virus vector, or any combination thereof, wherein AAV or rAAV is preferred.

65. The pharmaceutical composition of any one of claims 62-64, which is suitable for local administration to glial cells in one or more of the following sites: 1) glial cells in the striatum; ii) in the substantia nigra of the brain iii) glial cells in the retina; iv) glial cells in the inner ear; v) glial cells in the spinal cord; vi) glial cells in the prefrontal cortex; vii) glial cells in the motor cortex cells; viii) glial cells in the hypothalamus; and ix) glial cells in the ventral tegmental area (VTA).

66. The pharmaceutical composition of any one of claims 62-65, which is suitable for intracranial or intraocular administration.

67. The pharmaceutical composition of any one of claims 62-66, which further comprises i) one or more dopamine neuron-related factors, or ii) one or more retinal ganglion cell-associated factors used to express in Müller glial cells,
1) the one or more dopamine neuron-related factors are selected from: FoxA2, Lmx1a, Lmx1b, Nurr1, Pbxla, Pitx3, Gata2, Gata3, FGF8, BMP, En1, En2, PET1, Pax family proteins (Pax3, Pax6, etc.), SHH, Wnt family proteins, TGF-β family proteins, and any combination thereof;
2) the one or more retinal ganglion cell-related factors are selected from: β-catenin, Oct4, Sox2, Klf4, Crx, aCamKII, Brn3a, Brn3b, Brn3C, Math5, Otx2, Ngn2, Ngn1, AscL1, miRNA9, miRNA-124, Nr2e3, Nrl, and any combination thereof.

68. A medicine box or kit comprising the pharmaceutical composition of any one of claims 62-67.

69. A fusion protein comprising a DNA binding protein fused to one or more REST inhibitory domains.

70. A composition comprising a) a DNA binding protein and b) a protein comprising one or more REST inhibitory domains, wherein said a) and b) are capable of associating to form a protein complex.

71. The composition of claim 70, wherein said a) and b) are respectively linked to a pair of self-assembled assemblies, and said self-assembled assemblies can be combined with each other.

72. The composition of claim 71, wherein the pair of self-assembled assemblies can be selected from: i) two protein domains that can bind to each other (for example, an antigen and an antibody; or an antigen in an antigen and an antibody binding fragment); and ii) RNA splice donors and splice acceptors.

73. The fusion protein of claim 69 or the composition of claim 70, wherein the one or more REST inhibitory domains comprise the N-terminal inhibitory region of REST and/or the C-terminal inhibitory region of REST.

74. The fusion protein of claim 69 or the composition of claim 70, wherein the one or more REST inhibitory domains are derived from human REST protein or animal REST protein.

75. The fusion protein or composition of claim 73, wherein the N-terminal inhibitory region of REST comprises amino acids from 1 to 83 of REST or its fragment having transcriptional inhibitory activity.

76. The fusion protein or composition of claim 73 or 75, wherein the N-terminal inhibitory region of the REST comprises the sequence shown in SEQ ID NO: 16 or its fragment having transcriptional inhibitory activity.

77. The fusion protein or composition of claim 73, wherein the C-terminal inhibitory region of REST comprises amino acids from 1008 to 1097 of REST or its fragment having transcriptional inhibitory activity.

78. The fusion protein or composition of claim 73 or 77, wherein the C-terminal inhibitory region of the REST comprises the sequence shown in SEQ ID NO: 18 or its fragment having transcriptional inhibitory activity.

79. The fusion protein or composition of any one of claims 75-78, wherein the fragment having transcriptional repression activity comprises at least 20 consecutive amino acids, 30 consecutive amino acids, 40 consecutive amino acids, or 50 consecutive amino acids of the REST protein.

80. The fusion protein or composition of any one of claims 68-79, wherein the DNA binding protein is selected from sequence-guided DNA binding protein, transcription activator-like effector nuclease (TALEN), zinc finger nuclease (ZFN), or the DNA-binding portion of a transcription factor.

81. The fusion protein or composition of claim 80, wherein the sequence-guided DNA binding protein is a CRISPR-Cas protein or its variant thereof.

82. The fusion protein or composition of claim 81, wherein the CRISPR-Cas protein variant does not have nuclease activity.

83. The fusion protein or composition of any one of claims 80-82, wherein the sequence-guided DNA-binding protein is a Cas9 protein, or a Cas9 variant without nuclease activity.

84. The fusion protein or composition of any one of claims 80-82, wherein the sequence-guided DNA-binding protein is a Cas12 protein, or a Cas12 variant without nuclease activity.

85. The fusion protein or composition of any one of claims 73, 75 or 76, wherein at least one of said REST N-terminal inhibitory domains is linked to the N-terminal or C-terminal of said DNA binding protein.

86. The fusion protein or composition of any one of claims 73, 77 or 78, wherein at least one of said REST C-terminal inhibitory domains is linked to the N-terminal or C-terminal of said DNA binding protein.

87. The fusion protein or composition of any one of claims 73, 75 or 76, comprising at least one N-terminal inhibitory domain of REST connected in a tandem array to the N-terminus or C-terminus of said DNA binding protein.

88. The fusion protein or composition of any one of claims 73, 77 or 78, comprising at least one C-terminal inhibitory domain of REST connected in a tandem array to the N-terminal or C-terminal of said DNA binding protein.

89. The fusion protein or composition of any one of claims 69-88, comprising the amino acid sequence shown in SEQ ID NO: 25-27.

90. A polynucleotide encoding the fusion protein or composition of any one of claims 69-89.

91. The polynucleotide of claim 90 comprising a first polynucleotide fragment encoding a DNA binding protein and a second polynucleotide fragment encoding a protein comprising one or more REST inhibitory domains.

92. The polynucleotide of claim 91, wherein the first polynucleotide segment is linked to the second polynucleotide segment by a self-cleavable third nucleotide segment.

93. A method for inhibiting the expression of a target gene in a cell, comprising delivering the fusion protein or composition according to any one of claims 69-89, or the polynucleotide according to any one of claims 90-92, wherein the DNA binding protein can bind to the target gene or its regulatory sequence, and inhibit the expression of the target gene.

94. A method for inhibiting the expression of a target gene in a subject, comprising delivering the fusion protein or composition according to any one of claims 69-89, or the polynucleotide according to any one of claims 90-92, wherein the DNA binding protein can bind to the target gene or its regulatory sequence, and inhibit the expression of the target gene.

95. The method of claim 93 or 94, wherein the DNA-binding protein is selected from sequence-guided DNA-binding proteins, transcription activator-like effector nucleases (TALENs), zinc finger nuclease (ZFNs), or the DNA-binding portion of a transcription factor.

96. The method of any one of claims 95, wherein the sequence-guided DNA binding protein is a CRISPR-Cas protein or a variant thereof.

97. The method of claim 96, wherein the CRISPR-Cas protein variant does not have nuclease activity.

98. The method of any one of claims 95-97, wherein the sequence-guided DNA-binding protein is a Cas9 protein, or a Cas9 variant without nuclease activity.

99. The method of any one of claims 95-97, wherein the sequence-guided DNA-binding protein is Cas12 protein, or a Cas12 variant without nuclease activity.

100. The method of any one of claims 93-99, wherein said method further comprises delivering to the said cell or the said subject a guide RNA which comprises a targeting sequence complementary to a target region of a target gene or its regulatory sequence, and a sequence binding to the said DNA binding protein.
